# EUROPEAN PATENT APPLICATION

(11) **EP 2 604 694 A1**
(43) Date of publication of application: **19.06.2013**
(21) Application number: 11306664.1
(22) Date of filing: 14.12.2011
(51) Int. Cl.: C12N 15/82, A01H 5/10

(54) **Method for plant improvement**

(71) Applicant: Genoplante-Valor, 75015 Paris (FR); Biogemma, 75001 Paris (FR)
(72) Inventor: Paul, Wyatt, 63720 Chappes (FR); Rouster, Jacques, 63720 Chappes (FR)
(74) Representative: Flesselles, Bruno F.G.

(57) **Abstract**

The invention relates to the field of plant improvement, in particular of the improvement of yield for cereal plants, by down-regulation of the gene coding for Arf2.

<110>  BIOGEMMA
       GENOPLANTE-VALOR

<120>  METHOD FOR PLANT IMPROVEMENT

<130>  BRV 49 - EP PRIO

<160>  28

<170>  PatentIn version 3.3

<210>  1
<211>  801
<212>  PRT
<213>  Zea mays

<400>  1

Met Ala Leu Pro Ser Gln Ala Pro Ser Asn Ser Gly Asp Pro Leu Tyr
1               5                   10                  15

Pro Glu Leu Trp Arg Ala Cys Ala Gly Pro Leu Val Thr Val Pro Arg
            20                  25                  30

Val Gly Asp Leu Val Phe Tyr Phe Pro Gln Gly His Ile Glu Gln Val
        35                  40                  45

Glu Ala Ser Met Asn Gln Val Ala Gly Asn Pro Met Arg Leu Tyr Asp
    50                  55                  60

Leu Pro Ser Lys Leu Leu Cys Arg Val Leu Asn Val Glu Leu Lys Ala
65                  70                  75                  80

Glu Thr Asp Thr Asp Glu Val Tyr Ala Gln Ile Met Leu Met Pro Glu
                85                  90                  95

Pro Glu Gln Asn Asp Val Ala Ala Glu Lys Thr Ser Ser Gly Ser Ala
            100                 105                 110

Ala Pro Pro Arg Pro Ala Val Arg Ser Phe Cys Lys Thr Leu Thr Ala
        115                 120                 125

Ser Asp Thr Ser Thr His Gly Gly Phe Ser Val Leu Arg Arg His Ala
    130                 135                 140

Asp Glu Cys Leu Pro Ala Leu Asp Met Ser Gln Ser Pro Pro Thr Gln
145                 150                 155                 160

Glu Leu Val Ala Lys Asp Leu His Gly Met Glu Trp Arg Phe Arg His
                165                 170                 175

Ile Phe Arg Gly Gln Pro Arg Arg His Leu Leu Gln Ser Gly Trp Ser

180                 185                 190

Val Phe Val Ser Ser Lys Arg Leu Val Ala Gly Asp Ala Phe Ile Phe
        195                 200                 205

Leu Arg Gly Glu Asn Gly Glu Leu Arg Val Gly Val Arg Arg Ala Met
    210                 215                 220

Arg Gln Leu Ser Asn Val Pro Ser Ser Val Ile Ser Ser Gln Ser Met
225                 230                 235                 240

His Leu Gly Val Leu Ala Thr Ala Trp His Ala Ile Asn Thr Lys Ser
                245                 250                 255

Met Phe Thr Val Tyr Tyr Lys Pro Arg Thr Ser Pro Ser Glu Phe Ile
            260                 265                 270

Ile Pro Tyr Asp Gln Tyr Met Glu Ser Val Lys Asn Asn Tyr Ser Ile
        275                 280                 285

Gly Met Arg Phe Arg Met Arg Phe Glu Gly Glu Glu Ala Pro Glu Gln
    290                 295                 300

Arg Phe Thr Gly Thr Ile Val Gly Cys Glu Asn Leu Asp Pro Leu Trp
305                 310                 315                 320

Pro Asp Ser Ser Trp Arg Tyr Leu Lys Val Arg Trp Asp Glu Pro Ser
                325                 330                 335

Thr Ile Pro Arg Pro Asp Lys Val Ser Pro Trp Lys Ile Glu Pro Ala
            340                 345                 350

Ser Ser Pro Pro Val Asn Pro Leu Pro Leu Ser Arg Gly Lys Arg Pro
        355                 360                 365

Arg Gln Asn Ala Pro Pro Pro Ser Pro Glu Ser Ser Val Leu Thr Lys
    370                 375                 380

Glu Gly Ala Thr Lys Ile Asp Thr Asp Ser Ala Gln Thr Pro His Gln
385                 390                 395                 400

Asn Ser Val Leu Gln Ser Gln Glu Gln Met Ser Phe Arg Asn Asn Leu
                405                 410                 415

Thr Glu Ser Thr Asp Ser Asp Ser Thr Val Gln Lys Gln Met Met Trp
            420                 425                 430

Ser Pro Ser Pro Asn Gly Lys Val His Thr Asn Phe Gln Pro Arg Pro
        435                 440                 445

Ala Met Asp Asn Trp Met Pro Leu Gly Arg Arg Glu Thr Asp Phe Lys
    450                 455                 460

Asp Thr Arg Ser Ala Phe Lys Asp Ala Arg Thr Ala Ser Gln Ser Phe
465                 470                 475                 480

Gly Asp Thr Gln Gly Phe Phe Met Gln Ala Tyr Asp Asp Ser Arg His
                485                 490                 495

Arg Leu Ser Phe Asn Asn Gln Phe Gln Asp Gln Gly Ser Ala His Arg
            500                 505                 510

Phe Ala Asp Pro Tyr Phe Tyr Met Pro Gln Gln Pro Ser Leu Thr Val
        515                 520                 525

Glu Ser Gly Thr Arg Thr Gln Thr Ala Asn Asn Asp Leu Arg Phe Trp
    530                 535                 540

Ser Glu Arg Asn Ser Met Tyr Gly Asn Pro Ser Asp Gln Gln Gln Gly
545                 550                 555                 560

Phe Ser Phe Gly Gln Asn Thr Ser Ser Trp Leu Asn Gln Pro Leu Pro
                565                 570                 575

Gln Val Glu Gln Ser Arg Val Val Arg Pro His Ala Thr Val Ala Pro
            580                 585                 590

Phe Asp Leu Glu Lys Thr Arg Glu Gly Ser Gly Phe Lys Ile Phe Gly
        595                 600                 605

Phe Gln Val Asp Thr Thr Asn Pro Ser Pro Val Gln Leu Ser Ser Pro
    610                 615                 620

Leu Ser Ala Ile Arg Glu His Val Val Gln Thr Arg Pro Ser Ala Pro
625                 630                 635                 640

Val Asn Glu Leu Gln Pro Val Gln Ile Glu Cys Leu Pro Glu Val Ser
                645                 650                 655

Val Ser Thr Ala Gly Thr Ala Ala Glu Asn Ile Gln Gln Val Gln Gln
            660                 665                 670

Ser Ser Lys Asp Ile Gln Ser Lys Ser Gln Gly Ala Ser Thr Arg Ser
        675                 680                 685

Cys Thr Lys Val His Lys Gln Gly Val Ala Leu Gly Arg Ser Val Asp
    690                 695                 700

Leu Ser Lys Phe Thr Asp Tyr Gly Glu Leu Lys Ala Glu Leu Asp Lys
705                 710                 715                 720

Met Phe Glu Phe Glu Gly Glu Leu Val Ser Ala Asn Arg Asn Trp Gln
                725                 730                 735

Ile Val Tyr Thr Asp Asn Glu Gly Asp Met Met Leu Val Gly Asp Asp
            740                 745                 750

Pro Trp Glu Glu Phe Cys Asn Ile Val Arg Lys Ile Tyr Ile Tyr Thr
        755                 760                 765

Lys Glu Glu Val Gln Lys Met Asn Ser Lys Ser Ser Val Pro Arg Lys
    770                 775                 780

Glu Glu Pro Pro Ala Ala Gly Glu Gly Cys Ala Ala Ala Ala Ala Asn
785                 790                 795                 800

Glu

<210>  2
<211>  2406
<212>  DNA
<213>  Zea mays

<400>  2
atggcgctgc cgtcccaggc cccgtctaac tcaggggatc cgctgtaccc ggagctctgg      60

cgcgcctgcg ccggcccgct cgtcaccgtc ccccgcgtcg gcgacctcgt cttctacttc      120

ccccagggac acatcgagca ggtggaggcg tccatgaacc aggttgccgg gaacccgatg      180

cgtctctacg atctgccttc caagctgctc tgccgcgtgc tcaacgtcga gctgaaggcg      240

gagaccgaca ccgacgaggt gtacgcgcag atcatgctca tgccggagcc cgagcaaaac      300

gatgtggctg ccgagaagac gagctctggg tctgctgcgc caccgaggcc agcggtcagg      360

tcattctgca agacgctcac cgcctccgac accagcacac acggcggctt ctctgtcctg      420

cgccgccacg ctgacgagtg cctccctgcc ctggatatgt cccagtcgcc tcccactcag      480

gagctggtgg cgaaggatct gcatggcatg gagtggcgat tccgccacat cttccgcggg      540

caacccagga ggcatctcct tcagagtggt tggagtgttt ttgtcagttc caaaaggctt      600

gttgctggag acgccttcat tttcctcaga ggagaaaacg gtgaacttcg agttggtgtt      660

aggcgcgcta tgaggcagtt gtctaatgta ccttcttcgg ttatatctag ccaaagcatg      720

catctcgggg ttcttgcgac tgcatggcac gctattaaca caaagtccat gttcactgtc      780

tactacaaac ctaggacgag cccttcagag ttcatcattc catatgatca atatatggag      840

tcagtaaaaa acaattattc tattgggatg agattcagga tgaggtttga aggggaagag      900

gcccccgagc agaggtttac tggtacaata gttggctgtg aaaatcttga cccactatgg 960

cctgattcca gttggagata cttgaaggtg cgctgggatg agccttctac tatcccgcgg 1020

ccagataagg tctctccctg gaagatagag cctgcttcat cacctcctgt taatcctctc 1080

cctctttcta gaggaaaaag acctagacaa aatgcacctc caccttcccc tgaatcatct 1140

gttctcacaa aagaaggtgc aaccaagatt gatactgatt ctgctcaaac accacatcaa 1200

aattcggtct tgcaaagtca agagcagatg agctttagga acaacctgac tgaaagcacc 1260

gactctgatt ccactgttca gaagcagatg atgtggtccc catcacccaa tgggaaagtt 1320

cacactaatt ttcagccgag acctgctatg gataactgga tgccattggg aaggcgtgaa 1380

actgacttca aggacacccg ttctgccttc aaggatgccc gcaccgcttc tcaatcgttt 1440

ggagatacac aggggttctt tatgcaagct tatgatgaca gtcgtcaccg tctttctttc 1500

aacaatcagt ttcaagatca gggttcagct catcgctttg cagatccata cttctatatg 1560

cctcaacaac cttctctgac ggttgaatca ggcacaagga cgcaaacagc gaacaatgac 1620

ttgcgttttt ggagtgagcg gaattcgatg tatggtaatc caagtgacca acagcagggt 1680

ttcagctttg ggcaaaacac atcaagttgg ttgaaccagc cacttcccca ggttgaacag 1740

tcacgagtgg tcaggccgca tgcaacggtt gctccatttg atttagagaa gactagagaa 1800

ggcagtgggt tcaaaatatt tggtttccaa gttgatacaa ccaatccatc acctgtccaa 1860

ttgagctctc cattgtctgc tatccgagag catgtggtac aaactcgacc atcggcacca 1920

gtgaatgaat tgcaacctgt gcaaattgag tgcttgcctg aggtgtccgt aagcacagct 1980

ggaactgcag ctgagaacat tcagcaagtc cagcaaagtt cgaaagatat tcaaagcaag 2040

tcccagggcg cttcaacaag gagctgtaca aaggttcata agcaaggagt tgcgcttggc 2100

cggtccgtgg acctctctaa attcaccgac tacggtgagc tcaaagcgga actagacaag 2160

atgtttgagt tcgagggcga attggtatct gctaaccgga actggcagat cgtttatacc 2220

gacaacgagg gtgatatgat gcttgtcgga gatgacccgt gggaggagtt ctgcaacata 2280

gtgcgcaaga tctacatcta cacgaaggag gaggtccaga agatgaactc gaaatcatcc 2340

gtgccgagga aggaggaacc cccagcagca ggcgaaggct gcgccgccgc cgccgcaaac 2400

gagtag 2406

<210> 3
<211> 808
<212> PRT
<213> Oryza sativa

<400> 3

Met Pro Pro Ala Ala Met Ala Pro Pro Pro Pro Gln Gly Ser Ser
1               5                   10                  15

Thr Gly Asp Pro Leu Tyr Asp Glu Leu Trp His Ala Cys Ala Gly Pro

20                  25                  30

Leu Val Thr Val Pro Arg Val Gly Asp Leu Val Phe Tyr Phe Pro Gln
        35                  40                  45

Gly His Ile Glu Gln Val Glu Ala Ser Met Asn Gln Val Ala Asp Ser
    50                  55                  60

Gln Met Arg Leu Tyr Asp Leu Pro Ser Lys Leu Leu Cys Arg Val Leu
65                  70                  75                  80

Asn Val Glu Leu Lys Ala Glu Gln Asp Thr Asp Glu Val Tyr Ala Gln
                85                  90                  95

Val Met Leu Met Pro Glu Pro Glu Gln Asn Glu Met Ala Val Glu Lys
            100                 105                 110

Thr Thr Pro Thr Ser Gly Pro Val Gln Ala Arg Pro Pro Val Arg Ser
        115                 120                 125

Phe Cys Lys Thr Leu Thr Ala Ser Asp Thr Ser Thr His Gly Gly Phe
    130                 135                 140

Ser Val Leu Arg Arg His Ala Asp Glu Cys Leu Pro Pro Leu Asp Met
145                 150                 155                 160

Thr Gln Ser Pro Pro Thr Gln Glu Leu Val Ala Lys Asp Leu His Ser
                165                 170                 175

Met Asp Trp Arg Phe Arg His Ile Phe Arg Gly Gln Pro Arg Arg His
            180                 185                 190

Leu Leu Gln Ser Gly Trp Ser Val Phe Val Ser Ser Lys Arg Leu Val
        195                 200                 205

Ala Gly Asp Ala Phe Ile Phe Leu Arg Gly Glu Asn Gly Glu Leu Arg
    210                 215                 220

Val Gly Val Arg Arg Ala Met Arg Gln Leu Ser Asn Val Pro Ser Ser
225                 230                 235                 240

Val Ile Ser Ser Gln Ser Met His Leu Gly Val Leu Ala Thr Ala Trp
                245                 250                 255

His Ala Ile Asn Thr Lys Ser Met Phe Thr Val Tyr Tyr Lys Pro Arg
            260                 265                 270

Thr Ser Pro Ser Glu Phe Ile Ile Pro Tyr Asp Gln Tyr Met Glu Ser
        275                 280                 285

Val Lys Asn Asn Tyr Ser Val Gly Met Arg Phe Arg Met Arg Phe Glu
    290                 295                 300

Gly Glu Glu Ala Pro Glu Gln Arg Phe Thr Gly Thr Ile Ile Gly Ser
305                 310                 315                 320

Glu Asn Leu Asp Pro Val Trp Pro Glu Ser Ser Trp Arg Ser Leu Lys
                325                 330                 335

Val Arg Trp Asp Glu Pro Ser Thr Ile Pro Arg Pro Asp Arg Val Ser
            340                 345                 350

Pro Trp Lys Ile Glu Pro Ala Ser Ser Pro Pro Val Asn Pro Leu Pro
        355                 360                 365

Leu Ser Arg Val Lys Arg Pro Arg Pro Asn Ala Pro Pro Ala Ser Pro
    370                 375                 380

Glu Ser Pro Ile Leu Thr Lys Glu Ala Ala Thr Lys Val Asp Thr Asp
385                 390                 395                 400

Pro Ala Gln Ala Gln Arg Ser Gln Asn Ser Thr Val Leu Gln Gly Gln
                405                 410                 415

Glu Gln Met Thr Leu Arg Ser Asn Leu Thr Glu Ser Asn Asp Ser Asp
            420                 425                 430

Val Thr Ala His Lys Pro Met Met Trp Ser Pro Ser Pro Asn Ala Ala
        435                 440                 445

Lys Ala His Pro Leu Thr Phe Gln Gln Arg Pro Pro Met Asp Asn Trp
    450                 455                 460

Met Gln Leu Gly Arg Arg Glu Thr Asp Phe Lys Asp Val Arg Ser Gly
465                 470                 475                 480

Ser Gln Ser Phe Gly Asp Ser Pro Gly Phe Phe Met Gln Asn Phe Asp
                485                 490                 495

Glu Ala Pro Asn Arg Leu Thr Ser Phe Lys Asn Gln Phe Gln Asp Gln
            500                 505                 510

Gly Ser Ala Arg His Phe Ser Asp Pro Tyr Tyr Tyr Val Ser Pro Gln
        515                 520                 525

Pro Ser Leu Thr Val Glu Ser Ser Thr Gln Met His Thr Asp Ser Lys
    530                 535                 540

Glu Leu His Phe Trp Asn Gly Gln Ser Thr Val Tyr Gly Asn Ser Arg
545                 550                 555                 560

Asp Arg Pro Gln Asn Phe Arg Phe Glu Gln Asn Ser Ser Ser Trp Leu
                565                 570                 575

Asn Gln Ser Phe Ala Arg Pro Glu Gln Pro Arg Val Ile Arg Pro His
            580                 585                 590

Ala Ser Ile Ala Pro Val Glu Leu Glu Lys Thr Glu Gly Ser Gly Phe
        595                 600                 605

Lys Ile Phe Gly Phe Lys Val Asp Thr Thr Asn Ala Pro Asn Asn His
    610                 615                 620

Leu Ser Ser Pro Met Ala Ala Thr His Glu Pro Met Leu Gln Thr Pro
625                 630                 635                 640

Ser Ser Leu Asn Gln Leu Gln Pro Val Gln Thr Asp Cys Ile Pro Glu
                645                 650                 655

Val Ser Val Ser Thr Ala Gly Thr Ala Thr Glu Asn Glu Lys Ser Gly
            660                 665                 670

Gln Gln Ala Gln Gln Ser Ser Lys Asp Val Gln Ser Lys Thr Gln Val
        675                 680                 685

Ala Ser Thr Arg Ser Cys Thr Lys Val His Lys Gln Gly Val Ala Leu
    690                 695                 700

Gly Arg Ser Val Asp Leu Ser Lys Phe Ser Asn Tyr Asp Glu Leu Lys
705                 710                 715                 720

Ala Glu Leu Asp Lys Met Phe Glu Phe Asp Gly Glu Leu Val Ser Ser
                725                 730                 735

Asn Lys Asn Trp Gln Ile Val Tyr Thr Asp Asn Glu Gly Asp Met Met
            740                 745                 750

Leu Val Gly Asp Asp Pro Trp Glu Glu Phe Cys Ser Ile Val Arg Lys
        755                 760                 765

Ile Tyr Ile Tyr Thr Lys Glu Glu Val Gln Lys Met Asn Ser Lys Ser
    770                 775                 780

Asn Ala Pro Arg Lys Asp Asp Ser Ser Glu Asn Glu Lys Gly His Leu
785                 790                 795                 800

Pro Met Pro Asn Lys Ser Asp Asn
                805

<210>  4
<211>  801
<212>  PRT
<213>  Hordeum vulgare

<400>  4

Met Pro Pro Pro Ala Met Ala Thr Pro Gln Ala Pro Ser Ala Gly Asp
1               5                   10                  15

Pro Leu Tyr Asp Glu Leu Trp His Ala Cys Ala Gly Pro Leu Val Thr
            20                  25                  30

Val Pro Arg Val Gly Asp Leu Val Phe Tyr Phe Pro Gln Gly His Ile
        35                  40                  45

Glu Gln Val Glu Ala Ser Met Asn Gln Val Ala Gly Asn Gln Met Arg
    50                  55                  60

Leu Tyr Asp Leu Pro Pro Lys Leu Leu Cys Arg Val Ile Asn Val Glu
65                  70                  75                  80

Leu Lys Ala Glu Ala Asp Thr Asp Glu Val Tyr Ala Gln Val Met Leu
                85                  90                  95

Met Pro Glu Pro Glu Gln Asn Glu Met Ala Val Asp Lys Ser Thr Ser
            100                 105                 110

Thr Thr Gly Ala Thr Pro Pro Arg Pro Ala Val Arg Ser Phe Cys Lys
        115                 120                 125

Thr Leu Thr Ala Ser Asp Thr Ser Thr His Gly Gly Phe Ser Val Leu
    130                 135                 140

Arg Arg His Ala Asp Glu Cys Leu Pro Pro Leu Asp Met Thr Gln Ser
145                 150                 155                 160

Pro Pro Thr Gln Glu Leu Val Ala Lys Asp Leu His Gly Met Asp Trp
                165                 170                 175

Arg Phe Arg His Ile Phe Arg Gly Gln Pro Arg Arg His Leu Leu Gln
            180                 185                 190

Ser Gly Trp Ser Val Phe Val Ser Ser Lys Arg Leu Val Ala Gly Asp
        195                 200                 205

Ala Phe Ile Phe Leu Arg Gly Glu Ser Gly Glu Leu Arg Val Gly Val
    210                 215                 220

Arg Arg Ala Met Arg Gln Leu Ser Asn Val Pro Ser Ser Val Ile Ser
225                 230                 235                 240

Ser His Ser Met His Leu Gly Val Leu Ala Thr Ala Trp His Ala Ile
                245                 250                 255

Asn Thr Lys Ser Met Phe Thr Val Tyr Tyr Lys Pro Arg Thr Ser Pro
            260                 265                 270

Ser Glu Phe Ile Ile Pro Tyr Asp Gln Tyr Met Glu Ser Val Lys Asn
        275                 280                 285

Asn Tyr Ser Ile Gly Met Arg Phe Arg Met Arg Phe Glu Gly Glu Glu
    290                 295                 300

Ala Pro Glu Gln Arg Phe Thr Gly Thr Ile Val Gly Ser Glu Asn Leu
305                 310                 315                 320

Asp Gln Leu Trp Pro Glu Ser Asn Trp Arg Ser Leu Lys Val Arg Trp
                325                 330                 335

Asp Glu Pro Ser Thr Ile Pro Arg Pro Asp Arg Val Ser Pro Trp Lys
            340                 345                 350

Ile Glu Pro Ala Ser Ser Pro Pro Val Asn Pro Leu Pro Leu Ser Arg
        355                 360                 365

Val Lys Arg Pro Arg Pro Asn Val Pro Pro Val Ser Pro Glu Ser Ser
    370                 375                 380

Val Leu Thr Lys Glu Gly Ala Thr Lys Ile Asp Met Asp Ser Ala Gln
385                 390                 395                 400

Ala Gln Gln Arg Asn Gln Asn Ser Met Val Leu Gln Gly Gln Glu His
                405                 410                 415

Met Thr Leu Arg Thr Asn Asn Leu Thr Gly Ser Asn Asp Ser Asp Ala
            420                 425                 430

Thr Val Gln Lys Pro Met Met Trp Ser Pro Ser Pro Asn Ile Gly Lys
        435                 440                 445

Asn His Ala Ser Ala Phe Gln Gln Arg Pro Ser Met Glu Asn Trp Met
    450                 455                 460

Gln Leu Gly Arg Cys Glu Thr Ala Phe Lys Asp Ala Ser Ser Gly Ala
465                 470                 475                 480

Gln Ser Phe Gly Asp Ser Gln Gly Phe Phe Met Gln Thr Phe Asp Glu
                485                 490                 495

Ala Pro Asn Arg His Gly Ser Phe Lys Asn Gln Phe Gln Asp His Ser
            500                 505                 510

Ser Ala Arg His Phe Ser Asp Pro Tyr Thr Lys Met His Thr Glu Ala
        515                 520                 525

Asn Glu Phe His Phe Trp Asn Ser Gln Ser Thr Val Tyr Gly Asn Pro
    530                 535                 540

Arg Asp Gln Ser Gln Gly Phe Arg Phe Glu Glu His Pro Ser Asn Trp
545                 550                 555                 560

Leu Arg Gln Gln Gln Phe Ser Pro Val Glu Gln Pro Arg Val Ile Arg
                565                 570                 575

Pro Gln Ala Ser Ile Ala Pro Val Asp Leu Glu Lys Ala Arg Glu Gly
            580                 585                 590

Ser Gly Phe Lys Ile Phe Gly Phe Lys Val Asp Thr Thr Ser Ala Pro
        595                 600                 605

Ser Asn His Leu Ser Ser Thr Met Ala Val Ile His Glu Pro Val Leu
    610                 615                 620

Gln Thr Gln Ala Ser Ala Ser Leu Thr Gln Leu Gln His Ala His Ile
625                 630                 635                 640

Asp Cys Ile Pro Glu Leu Ser Val Ser Thr Ala Gly Thr Thr Glu Asn
                645                 650                 655

Glu Lys Ser Ile Gln Gln Ala Pro Asn Ser Ser Lys Asp Val Gln Ser
            660                 665                 670

Lys Ser His Gly Ala Ser Thr Arg Ser Cys Thr Lys Val His Lys Gln
        675                 680                 685

Gly Val Ala Leu Gly Arg Ser Val Asp Leu Ser Lys Phe Gly Asp Tyr
    690                 695                 700

Asp Glu Leu Thr Ala Glu Leu Asp Arg Met Phe Glu Phe Asp Gly Glu
705                 710                 715                 720

Leu Met Ser Ser Asn Arg Asp Trp Gln Ile Val Tyr Thr Asp Pro Glu
                725                 730                 735

Gly Asp Met Met Leu Val Gly Asp Asp Pro Trp Glu Glu Phe Cys Ser
            740                 745                 750

Ile Val Arg Lys Ile Phe Ile Tyr Thr Lys Glu Glu Val Gln Lys Met
        755                 760                 765

Asn Ser Lys Ser Ser Thr Pro Arg Lys Glu Glu Gly Ser Ala Asp Ala
    770                 775                 780

Asp Gly Ala Asn Glu Lys Ala His Leu Ala Ala Ser Ser His Leu Asp
785                 790                 795                 800

Asn

<210>  5
<211>  704
<212>  PRT
<213>  Sorghum bicolor

<400>  5

Met Pro Pro Ala Ala Ala Met Ala Leu Pro Ser Gln Ala Pro Ser Asn
1               5                   10                  15

Ser Gly Asp Pro Leu Tyr Pro Glu Leu Trp Arg Ala Cys Ala Gly Pro
            20                  25                  30

Leu Val Thr Val Pro Arg Val Gly Asp Leu Val Phe Tyr Phe Pro Gln
        35                  40                  45

Gly His Ile Glu Gln Val Glu Ala Ser Met Asn Gln Val Ala Gly Asn
    50                  55                  60

Gln Met Arg Leu Tyr Asp Leu Pro Ser Lys Leu Leu Cys Arg Val Leu
65                  70                  75                  80

Asn Val Glu Leu Lys Ala Glu Thr Asp Thr Asp Glu Val Tyr Ala Gln
                85                  90                  95

Ile Met Leu Met Pro Glu Pro Glu Gln Thr Asp Val Ala Ala Glu Lys
            100                 105                 110

Ala Ser Ser Ala Ser Ala Ala Ser Pro Arg Pro Ala Val Arg Ser Phe
        115                 120                 125

Cys Lys Thr Leu Thr Ala Ser Asp Thr Ser Thr His Gly Gly Phe Ser
    130                 135                 140

Val Leu Arg Arg His Ala Asp Glu Cys Leu Pro Pro Leu Asp Met Thr
145                 150                 155                 160

Gln Ser Pro Pro Thr Gln Glu Leu Val Ala Lys Asp Leu His Gly Met
                165                 170                 175

Glu Trp Arg Phe Arg His Ile Phe Arg Gly Gln Pro Arg Arg His Leu
            180                 185                 190

Leu Gln Ser Gly Trp Ser Val Phe Val Ser Ser Lys Arg Leu Val Ala
        195                 200                 205

Gly Asp Ala Phe Ile Phe Leu Arg Gly Glu Asn Gly Glu Leu Arg Val
    210                 215                 220

Gly Val Arg Arg Ala Met Arg Gln Leu Ser Asn Val Pro Ser Ser Val
225                 230                 235                 240

Ile Ser Ser Gln Ser Met His Leu Gly Val Leu Ala Thr Ala Trp His
                245                 250                 255

Ala Ile Asn Thr Lys Ser Met Phe Thr Val Tyr Tyr Lys Pro Arg Thr
            260                 265                 270

Ser Pro Ser Glu Phe Ile Ile Pro Tyr Asp Gln Tyr Met Glu Ser Val
        275                 280                 285

Lys Asn Asn Tyr Ser Ile Gly Met Arg Phe Arg Met Arg Phe Glu Gly
    290                 295                 300

Glu Glu Ala Pro Glu Gln Arg Phe Thr Gly Thr Ile Val Gly Cys Glu
305                 310                 315                 320

Asn Leu Asp Pro Leu Trp Pro Asp Ser Ser Trp Arg Tyr Leu Lys Val
                325                 330                 335

Arg Trp Asp Glu Pro Ser Thr Ile Pro Arg Pro Asp Arg Val Ser Pro
            340                 345                 350

Trp Lys Ile Glu Pro Ala Ser Ser Pro Pro Val Asn Pro Leu Pro Leu
        355                 360                 365

Ser Ser Arg Val Lys Arg Pro Arg Gln Asn Ala Pro Pro Pro Ser Pro
    370                 375                 380

Glu Ala Ser Val Leu Thr Lys Glu Ser Ala Ala Lys Ile Asp Ile Asp
385                 390                 395                 400

Ser Ala Gln Thr Gln His Gln Asn Ser Val Leu Gln Gly Gln Glu Gln
                405                 410                 415

Met Thr Leu Arg Asn Asn Leu Thr Glu Ser Asn Asp Ser Asp Ser Thr
            420                 425                 430

Val Gln Lys Pro Met Met Trp Ser Pro Ser Pro Asn Gly Lys Ala His
        435                 440                 445

Thr Asn Phe Gln Gln Arg Pro Ala Met Asp Ser Trp Met Pro Met Gly
    450                 455                 460

Arg Arg Glu Thr Asp Phe Lys Asp Ser Arg Ser Ala Phe Lys Asp Ala
465                 470                 475                 480

Arg Thr Ala Ser Gln Ser Phe Arg Asp Thr Gln Gly Phe Phe Met Gln
                485                 490                 495

Ala Tyr Asp Asp Asn His His Arg Leu Ser Phe Asn Asn Gln Phe Gln
            500                 505                 510

Asp Gln Gly Ser Ala His Arg Phe Ala Asp Pro Tyr Phe Tyr Met Ala
        515                 520                 525

Gln Gln Pro Ser Leu Thr Val Glu Ser Ser Thr Arg Thr Gln Thr Ala
    530                 535                 540

Asn Asn Asp Leu Arg Phe Trp Gly Asp Gln Asn Ser Ile Tyr Gly Asn
545                 550                 555                 560

Pro Gly Asp Gln Gln Gln Gln Gly Phe Ser Phe Gly Gln Asn Pro Ser
                565                 570                 575

Ser Trp Leu Asn Gln Pro Phe Pro Gln Val Glu Gln Pro Arg Val Val
            580                 585                 590

Arg Pro His Ala Thr Val Ala Pro Phe Asp Leu Glu Lys Thr Arg Glu
        595                 600                 605

Gly Ser Gly Phe Lys Ile Phe Gly Phe Gln Val Asp Thr Thr Ser Pro
    610                 615                 620

Ser Pro Ala Gln Leu Ser Ser Pro Leu Cys Ala Ile Arg Glu His Val
625                 630                 635                 640

Val Gln Thr Arg Pro Ser Ala Pro Val Asn Glu Leu Gln Pro Val Gln
                645                 650                 655

Asn Glu Cys Leu Pro Glu Gly Ser Val Ser Thr Ala Gly Thr Ala Thr
            660                 665                 670

Glu Asn Glu Lys Asn Ile Gln Gln Ala Gln Pro Ser Ser Lys Asp Ile
        675                 680                 685

Gln Ser Lys Ser Gln Gly Ala Ser Thr Arg Ser Cys Thr Lys Val Val
    690                 695                 700

<210> 6
<211> 809
<212> PRT
<213> Brachypodium distachyon

<400> 6

Met Ala Pro Pro Pro Gln Gly Pro Ser Ala Gly Asp Pro Leu Phe Asn
1               5                   10                  15

Glu Leu Trp His Ala Cys Ala Gly Pro Leu Val Thr Val Pro Arg Val
            20                  25                  30

Gly Asp Leu Val Phe Tyr Phe Pro Gln Gly His Ile Glu Gln Val Glu
        35                  40                  45

Ala Ser Met Asn Gln Val Ala Asp Asn Gln Met Arg Leu Tyr Asp Leu
    50                  55                  60

Pro Ser Lys Leu Leu Cys Ser Val Ile Asn Val Glu Leu Lys Ala Glu
65                  70                  75                  80

Ala Asp Thr Asp Glu Val Tyr Ala Gln Val Met Leu Ile Pro Glu Asn
                85                  90                  95

Asp Gln Asn Glu Met Ala Val Glu Lys Ser Ser Ser Lys Ala Ala Thr
            100                 105                 110

Thr Leu Ala Lys Pro Ala Val Arg Ser Phe Cys Lys Thr Leu Thr Ala
        115                 120                 125

Ser Asp Thr Ser Thr His Gly Gly Phe Ser Val Leu Arg Arg His Ala
    130                 135                 140

Asp Glu Cys Leu Pro Pro Leu Asp Met Thr Gln Ser Pro Pro Thr Gln
145                 150                 155                 160

Glu Leu Val Ala Lys Asp Leu His Gly Met Asp Trp Arg Phe Arg His
                165                 170                 175

Ile Phe Arg Gly Gln Pro Arg Arg His Leu Leu Gln Ser Gly Trp Ser
            180                 185                 190

Val Phe Val Ser Ser Lys Arg Leu Val Ala Gly Asp Ala Phe Ile Phe
        195                 200                 205

Leu Arg Gly Glu Ser Gly Glu Leu Arg Val Gly Val Arg Arg Ala Met
    210                 215                 220

Arg Gln Leu Ser Asn Val Pro Ser Ser Val Ile Ser Ser His Ser Met
225                 230                 235                 240

His Leu Gly Val Leu Ala Thr Ala Trp His Ala Ile Asn Thr Lys Ser
                245                 250                 255

Met Phe Thr Val Tyr Tyr Lys Pro Arg Thr Ser Pro Ser Glu Phe Ile
            260                 265                 270

Ile Pro Tyr Asp Gln Tyr Met Glu Ser Val Lys Asn Asn Tyr Ser Ile
        275                 280                 285

Gly Val Arg Phe Arg Met Arg Phe Glu Gly Glu Glu Ala Pro Glu Gln
    290                 295                 300

Arg Phe Thr Gly Thr Ile Ile Gly Ser Glu Asn Leu Asp Pro Leu Trp
305                 310                 315                 320

Pro Glu Ser Ser Trp Arg Ser Leu Lys Val Arg Trp Asp Glu Pro Ser
                325                 330                 335

Thr Ile Pro Arg Pro Asp Arg Val Ser Pro Trp Lys Ile Glu Pro Ala
            340                 345                 350

Ser Ser Pro Pro Val Asn Pro Leu Pro Leu Ser Arg Val Lys Arg Pro
        355                 360                 365

Arg Pro Asn Val Pro Pro Ala Ser Pro Glu Ser Ser Ala Leu Thr Lys
    370                 375                 380

Glu Gly Ala Thr Lys Val Asp Val Asp Ser Ala Gln Ala Gln Arg Asn
385                 390                 395                 400

Gln Thr Ser Met Val Leu Gln Gly Gln Glu Pro Met Thr Leu Arg Ser
                405                 410                 415

Asn Asn Leu Thr Asp Ser Asn Asp Ser Asp Ala Thr Val Gln Lys Pro
            420                 425                 430

Met Met Trp Ser Pro Ser Pro Asn Ile Gly Lys Ser Arg Pro Leu Thr
        435                 440                 445

Phe Gln Gln Arg Pro Ser Met Asp Asn Trp Met Gln Leu Gly Arg Arg
    450                 455                 460

Glu Thr Asp Tyr Lys Asp Ala Ser Ser Gly Ala Gln Ser Phe Gly Asp
465                 470                 475                 480

Ser Pro Gly Phe Phe Val Gln Thr Tyr Asp Glu Ala Pro Asn His Leu
                485                 490                 495

Ala Ser Phe Lys Asn Gln Phe Gln Asp Gln Gly Pro Ala Arg His Phe
            500                 505                 510

Ser Glu Pro Tyr Phe Phe Met His Gln Gln Pro Asn Leu Thr Val Asp
        515                 520                 525

Ser Ser Thr Lys Met His Pro Glu Thr Asn Glu Leu His Phe Trp Asn
    530                 535                 540

Gly Gln Asn Thr Val Tyr Gly His Ser Gly Asp Gln Ser Gln Gly Phe
545                 550                 555                 560

Arg Phe Glu Glu His Pro Ser Asn Trp Ser Ser Gln Gln Phe Ser Arg
                565                 570                 575

Val Glu Gln Pro Arg Val Ile Arg Pro His Ala Ser Ile Ala Pro Val
            580                 585                 590

Asp Leu Glu Lys Thr Arg Glu Gly Ser Gly Phe Lys Ile Phe Gly Phe
        595                 600                 605

Lys Val Asp Thr Ala Ser Ala Pro Thr Asn His Leu Ser Ser Pro Met
    610                 615                 620

Ala Ala Thr His Glu Pro Ala Leu Gln Thr Gln Pro Ser Ala Ser Leu
625                 630                 635                 640

Asn Gln Leu Gln His Ala Gln Thr Asp Cys Phe Pro Glu Val Ser Val
                645                 650                 655

Ser Thr Gly Gly Thr Asn Glu Asn Glu Lys Ser Ile Gln Gln Ala Pro
            660                 665                 670

Gln Ser Ser Lys Asp Val Gln Ser Lys Ser His Gly Ala Ser Thr Arg
        675                 680                 685

Ser Cys Thr Lys Val His Lys Gln Gly Val Ala Leu Gly Arg Ser Val
    690                 695                 700

Asp Leu Ser Lys Phe Ser Asp Tyr Asp Glu Leu Lys Ala Glu Leu Asp
705                 710                 715                 720

Lys Met Phe Glu Phe Asp Gly Glu Leu Met Ser Ser Asn Lys Asn Trp
                725                 730                 735

Gln Ile Val Tyr Thr Asp Asn Glu Asp Asp Met Met Leu Val Gly Asp
            740                 745                 750

Asp Pro Trp Gly Glu Phe Cys Ser Ile Val Arg Lys Ile Cys Ile Tyr
        755                 760                 765

Thr Lys Glu Glu Val Gln Lys Met Asn Ser Lys Leu Ser Ala Pro Arg
    770                 775                 780

Lys Glu Asp Gly Ala Ala Pro Cys Asp Gly Ala Asn Glu Asn Ala Ala
785                 790                 795                 800

Leu Pro Ala Pro Gly Asn Leu Asp Asn
                805

<210>  7
<211>  742
<212>  PRT
<213>  Triticum aestivum

<400>  7

Met Asn Gln Val Ala Gly Asn Gln Met Arg Leu Tyr Asp Leu Pro Ser
1               5                   10                  15

Lys Leu Leu Cys Arg Val Ile Asn Val Glu Leu Lys Ala Glu Ala Asp
            20                  25                  30

Thr Asp Glu Val Tyr Ala Gln Val Met Leu Met Pro Glu Pro Glu Gln
        35                  40                  45

Asn Glu Met Ala Val Asp Lys Ser Thr Ser Thr Thr Gly Ala Thr Pro
    50                  55                  60

Pro Arg Pro Ala Val Arg Ser Phe Cys Lys Thr Leu Thr Ala Ser Asp
65                  70                  75                  80

Thr Ser Thr His Gly Gly Phe Ser Val Leu Arg Arg His Ala Asp Glu
                85                  90                  95

Cys Leu Pro Pro Leu Asp Met Thr Gln Ser Pro Pro Thr Gln Glu Leu
            100                 105                 110

Val Ala Lys Asp Leu His Gly Met Asp Trp Arg Phe Arg His Ile Phe
        115                 120                 125

Arg Gly Gln Pro Arg Arg His Leu Leu Gln Ser Gly Trp Ser Val Phe
    130                 135                 140

Val Ser Ser Lys Arg Leu Val Ala Gly Asp Ala Phe Ile Phe Leu Arg
145                 150                 155                 160

Gly Glu Ser Gly Glu Leu Arg Val Gly Val Arg Arg Ala Met Arg Gln
                165                 170                 175

Leu Ser Asn Val Pro Ser Ser Val Ile Ser Ser His Ser Met His Leu
            180                 185                 190

Gly Val Leu Ala Thr Ala Trp His Ala Ile Asn Thr Lys Ser Met Phe
        195                 200                 205

Thr Val Tyr Tyr Lys Pro Arg Thr Ser Pro Ser Glu Phe Ile Ile Pro
    210                 215                 220

Tyr Asp Gln Tyr Met Glu Ser Val Lys Asn Asn Tyr Ser Ile Gly Met
225                 230                 235                 240

Arg Phe Arg Met Arg Phe Glu Gly Glu Glu Ala Pro Glu Gln Arg Phe
                245                 250                 255

Thr Gly Thr Ile Val Gly Ser Glu Asn Leu Asp Gln Leu Trp Pro Glu
            260                 265                 270

Ser Asn Trp Arg Ser Leu Lys Val Arg Trp Asp Glu Pro Ser Thr Ile
        275                 280                 285

Pro Arg Pro Asp Arg Val Ser Pro Trp Lys Ile Glu Pro Ala Ser Ser
    290                 295                 300

Pro Pro Val Asn Pro Leu Pro Leu Ser Arg Val Lys Arg Pro Arg Pro
305                 310                 315                 320

Asn Val Pro Pro Val Ser Pro Glu Ser Ser Val Leu Thr Lys Glu Gly
                325                 330                 335

Ala Thr Lys Ile Asp Met Asp Ser Ala Gln Ala Gln Gln Arg Asn Gln
            340                 345                 350

Asn Ser Met Val Leu Gln Gly Gln Glu His Met Thr Leu Arg Thr Asn
        355                 360                 365

Asn Leu Thr Gly Ser Asn Glu Ser Asp Ala Thr Val Gln Lys Pro Met
    370                 375                 380

Met Trp Ser Pro Ser Pro Asn Ile Gly Lys Asn His Ala Ser Ala Phe
385                 390                 395                 400

Gln Gln Arg Pro Ser Met Asp Asn Trp Met Gln Leu Gly Arg Cys Asp
                405                 410                 415

Ala Ser Ser Gly Ala Gln Ser Phe Gly Asp Ser Gln Gly Phe Phe Met
            420                 425                 430

Gln Thr Phe Asp Glu Ala Pro Asn Arg His Gly Ser Phe Lys Asn Gln
        435                 440                 445

Phe Gln Asp His Ser Ser Ala Arg His Phe Ser Asp Pro Tyr Thr Lys
    450                 455                 460

Met Gln Thr Glu Ala Asn Glu Phe His Phe Trp Asn Ser Gln Ser Thr
465                 470                 475                 480

Val Tyr Gly Asn Pro Arg Asp Gln Ser Gln Gly Phe Arg Phe Glu Glu
                485                 490                 495

His Pro Ser Asn Trp Leu Arg Gln Gln Gln Leu Ser Pro Val Glu Gln
            500                 505                 510

Pro Arg Val Ile Arg Pro His Ala Ser Ile Ala Pro Val Asp Leu Glu
        515                 520                 525

Lys Thr Arg Glu Gly Ser Gly Phe Lys Ile Phe Gly Phe Lys Val Asp
    530                 535                 540

Thr Thr Ser Ala Pro Ser Asn His Leu Ser Ser Thr Thr Ala Ala Ile
545                 550                 555                 560

His Glu Pro Val Leu Gln Thr Gln Ala Ser Ala Ser Leu Thr Gln Leu
                565                 570                 575

Gln His Ala His Ala Asp Cys Ile Pro Glu Leu Ser Val Ser Thr Ala
            580                 585                 590

Gly Thr Thr Glu Asn Glu Lys Ser Ile Gln Gln Thr Pro His Ser Ser
        595                 600                 605

Lys Asp Val Gln Ser Lys Ser His Gly Ala Ser Thr Arg Ser Cys Thr
    610                 615                 620

Lys Val His Lys Gln Gly Val Ala Leu Gly Arg Ser Val Asp Leu Ser
625                 630                 635                 640

Lys Phe Gly Asp Tyr Asp Glu Leu Thr Ala Glu Leu Asp Arg Met Phe
                645                 650                 655

Glu Phe Asp Gly Glu Leu Met Ser Ser Asn Lys Asp Trp Gln Ile Val
            660                 665                 670

Tyr Thr Asp Pro Glu Gly Asp Met Met Leu Val Gly Asp Asp Pro Trp
        675                 680                 685

Glu Glu Phe Cys Asn Ile Val Arg Lys Ile Phe Ile Tyr Thr Lys Glu
    690                 695                 700

Glu Val Gln Lys Met Asn Ser Lys Ser Ser Thr Pro Arg Lys Glu Glu
705                 710                 715                 720

Gly Ser Ala Asp Ala Asp Gly Ala Asn Glu Lys Ala His Leu Ala Thr
                725                 730                 735

Ser Ser His Leu Asp Asn
            740

<210>  8
<211>  796
<212>  PRT
<213>  Triticum aestivum

<400>  8

Met Pro Pro Ala Ala Met Ala Pro Pro Gln Ala Pro Ser Ala Gly Asp
1               5                   10                  15

Pro Leu Tyr Asp Glu Leu Trp His Ala Cys Ala Gly Pro Leu Val Thr
            20                  25                  30

Val Pro Arg Val Gly Asp Leu Val Phe Tyr Phe Pro Gln Gly His Ile
        35                  40                  45

Glu Gln Val Glu Ala Ser Met Asn Gln Ile Ala Gly Asn Gln Met Arg
    50                  55                  60

Leu Tyr Asp Leu Pro Ser Lys Leu Leu Cys Arg Val Ile Asn Val Glu
65                  70                  75                  80

Leu Lys Ala Glu Ala Asp Thr Asp Glu Val Tyr Ala Gln Val Met Leu
                85                  90                  95

Met Pro Glu Pro Glu Gln Asn Glu Met Ala Val Asp Lys Ser Thr Ser
            100                 105                 110

Thr Thr Gly Ala Thr Pro Pro Arg Pro Ala Val Arg Ser Phe Cys Lys
        115                 120                 125

Thr Leu Thr Ala Ser Asp Thr Ser Thr His Gly Gly Phe Ser Val Leu
    130                 135                 140

Arg Arg His Ala Asp Glu Cys Leu Pro Pro Leu Asp Met Thr Gln Ser
145                 150                 155                 160

Pro Pro Thr Gln Glu Leu Val Ala Lys Asp Leu His Gly Met Asp Trp
                165                 170                 175

Arg Phe Arg His Ile Phe Arg Gly Gln Pro Arg Arg His Leu Leu Gln
            180                 185                 190

Ser Gly Trp Ser Val Phe Val Ser Ser Lys Arg Leu Val Ala Gly Asp
        195                 200                 205

Ala Phe Ile Phe Leu Arg Gly Glu Ser Gly Glu Leu Arg Gly Val Arg
    210                 215                 220

Arg Ala Met Arg Gln Leu Ser Asn Val Pro Ser Ser Val Ile Ser Ser
225                 230                 235                 240

His Ser Met His Leu Gly Val Leu Ala Thr Ala Trp His Ala Ile Asn
                245                 250                 255

Thr Lys Ser Met Phe Thr Val Tyr Tyr Lys Pro Arg Thr Ser Pro Ser
            260                 265                 270

Glu Phe Ile Ile Pro Tyr Asp Gln Tyr Met Glu Ser Val Lys Asn Asn
        275                 280                 285

Tyr Ser Ile Gly Met Arg Phe Arg Met Arg Phe Glu Gly Glu Glu Ala
    290                 295                 300

Pro Glu Gln Arg Leu Thr Gly Thr Ile Val Gly Ser Glu Asn Leu Asp
305                 310                 315                 320

Gln Leu Trp Pro Glu Ser Asn Trp Arg Ser Leu Lys Val Arg Trp Asp
                325                 330                 335

Glu Pro Ser Thr Ile Pro Arg Pro Asp Arg Val Ser Pro Trp Lys Ile
            340                 345                 350

Glu Pro Ala Ser Ser Pro Pro Val Asn Pro Leu Pro Leu Ser Arg Val
        355                 360                 365

Lys Arg Pro Arg Pro Asn Val Pro Pro Val Ser Pro Glu Ser Ser Val
    370                 375                 380

Leu Thr Lys Glu Val Ala Ala Lys Ile Asp Met Asp Ser Ala Gln Ala
385                 390                 395                 400

Gln Gln Arg Asn Gln Asn Asn Met Val Leu Gln Gly Gln Glu His Met
                405                 410                 415

Thr Leu Arg Thr Asn Asn Leu Thr Ala Ser Asn Glu Ser Asp Ala Thr
            420                 425                 430

Val Gln Lys Pro Met Met Trp Ser Pro Ser Pro Asn Ile Gly Lys Asn
        435                 440                 445

His Ala Ser Ala Phe Gln Gln Arg Pro Ser Met Asp Asn Trp Met Gln
    450                 455                 460

Leu Gly Arg Cys Asp Ala Ser Ser Gly Ala Gln Ser Phe Gly Asp Ser
465                 470                 475                 480

Gln Gly Phe Phe Met Gln Thr Phe Asp Glu Ala Pro Asn Arg His Gly
                485                 490                 495

Ser Phe Lys Asn Gln Phe Gln Asp His Ser Ser Ala Arg His Phe Ser
            500                 505                 510

Asp Pro Tyr Thr Lys Met Gln Thr Glu Ala Ser Glu Phe His Phe Trp
        515                 520                 525

Asn Ser Gln Ser Thr Val Tyr Gly Asn Pro Arg Asp Gln Ser Gln Gly
    530                 535                 540

Phe Arg Phe Glu Glu His Pro Ser Asn Trp Leu Arg Gln Gln Gln Gln
545                 550                 555                 560

Phe Ser Pro Val Glu Gln Pro Arg Val Ile Arg Pro His Ala Ser Ile
                565                 570                 575

Ala Pro Val Asp Leu Glu Lys Thr Arg Glu Gly Ser Gly Phe Lys Ile
            580                 585                 590

Phe Gly Phe Lys Val Asp Thr Thr Ser Ala Pro Ser Asn His Leu Ser
        595                 600                 605

Ser Thr Met Ala Ala Ile His Glu Pro Val Leu Gln Thr Gln Ala Ser
    610                 615                 620

Ala Ser Leu Thr Gln Leu Gln His Ala His Ala Asp Cys Ile Pro Glu
625                 630                 635                 640

Leu Ser Val Ser Thr Ala Gly Thr Thr Glu Asn Glu Lys Ser Ile Gln
                645                 650                 655

Gln Ala Pro His Ser Ser Lys Asp Val Gln Ser Lys Ser His Gly Ala
            660                 665                 670

Ser Thr Arg Ser Cys Thr Lys Val His Lys Gln Gly Val Ala Leu Gly
        675                 680                 685

Arg Ser Val Asp Leu Ser Lys Phe Gly Asp Tyr Asp Glu Leu Thr Ala
    690                 695                 700

Glu Leu Asp Arg Met Phe Glu Phe Asp Gly Glu Leu Met Ser Ser Asn
705                 710                 715                 720

Lys Asp Trp Gln Ile Val Tyr Thr Asp Pro Glu Gly Asp Met Met Leu
                725                 730                 735

Val Gly Asp Asp Pro Trp Glu Glu Phe Cys Asn Ile Val Arg Lys Ile
            740                 745                 750

Phe Ile Tyr Thr Lys Glu Glu Val Gln Lys Met Asn Ser Lys Ser Ser
        755                 760                 765

Thr Pro Arg Lys Glu Glu Gly Ser Ala Asp Ala Asp Gly Ala Asn Glu
    770                 775                 780

Lys Ala His Pro Ala Thr Ser Ser His Leu Asp Asn
785                 790                 795

<210>  9
<211>  824
<212>  PRT
<213>  Triticum aestivum

<400>  9

Met Pro Pro Ala Ala Met Pro Pro Pro Pro Pro Pro Pro Gln Gly Ala
1               5                   10                  15

Ser Thr Gly Asp Pro Leu Tyr Asp Glu Leu Trp His Ala Cys Ala Gly
            20                  25                  30

Pro Leu Val Thr Val Pro Arg Val Gly Asp Met Val Tyr Tyr Phe Pro
        35                  40                  45

Gln Gly His Ile Glu Gln Val Glu Ala Ser Met Asn Gln Val Ala Ala
    50                  55                  60

Asn Gln Met Arg Leu Tyr Asp Leu Pro Ser Lys Leu Leu Cys Arg Val
65                  70                  75                  80

Leu Asn Val Glu Leu Lys Ala Glu Ala Asp Thr Asp Glu Val Tyr Ala
                85                  90                  95

Gln Val Met Leu Met Pro Glu Pro Glu Gln Ser Glu Thr Thr Thr Glu
            100                 105                 110

Lys Ser Ser Pro Ala Thr Gly Gly Ala Thr Pro Ala Arg Pro Ala Val
        115                 120                 125

Arg Ser Phe Cys Lys Thr Leu Thr Ala Ser Asp Thr Ser Thr His Gly
    130                 135                 140

Gly Phe Ser Val Leu Arg Arg His Ala Asp Glu Cys Leu Pro Pro Leu
145                 150                 155                 160

Asp Met Thr Gln Ser Pro Pro Thr Gln Glu Leu Val Ala Lys Asp Leu
                165                 170                 175

His Gly Met Glu Trp Arg Phe Arg His Ile Phe Arg Gly Gln Pro Arg
            180                 185                 190

Arg His Leu Leu Gln Ser Gly Trp Ser Val Phe Val Ser Ser Lys Arg
        195                 200                 205

Leu Val Ala Gly Asp Ala Phe Ile Phe Leu Arg Gly Glu Ser Gly Glu
    210                 215                 220

Leu Arg Val Gly Val Arg Arg Ala Met Arg Gln Leu Ser Asn Ile Ala
225                 230                 235                 240

Ser Ser Val Ile Ser Ser His Ser Met His Leu Gly Val Leu Ala Thr
                245                 250                 255

Ala Trp His Ala Ile Asn Thr Lys Thr Met Phe Thr Val Tyr Tyr Lys
            260                 265                 270

Pro Arg Thr Ser Arg Ser Glu Phe Ile Ile Pro Tyr Asp Lys Tyr Met
        275                 280                 285

Glu Ser Val Lys Asn Ile Tyr Ser Ile Gly Thr Arg Phe Lys Met Arg
    290                 295                 300

Phe Glu Gly Glu Glu Ala Pro Glu Gln Arg Phe Thr Gly Thr Ile Val
305                 310                 315                 320

Gly Ser Asp Asn Leu Asp Gln Leu Trp Pro Glu Ser Ser Trp Arg Ser
                325                 330                 335

Leu Lys Val Arg Trp Asp Glu Ser Ser Thr Ile Pro Arg Pro Asp Arg
            340                 345                 350

Val Ser Pro Trp Glu Ile Glu Pro Ala Ser Ser Pro Pro Val Asn Pro
        355                 360                 365

Leu Pro Ile Ser Arg Pro Lys Arg Ser Arg Pro Asn Val Pro Pro Ala
    370                 375                 380

Ser Pro Glu Ser Ser Val Arg Thr Lys Glu Gly Ala Thr Lys Ala Asp
385                 390                 395                 400

Met Asp Cys Ala Gln Ala Gln Arg Asn Gln Asn Asn Thr Ala Leu Pro
                405                 410                 415

Gly Gln Glu Gln Arg Ser Asn Lys Leu Thr Asp Ile Asn Asp Phe Asp
            420                 425                 430

Ala Ser Val Gln Lys Pro Met Met Trp Ser Pro Pro Pro Pro Pro Asn
        435                 440                 445

Ile Gly Lys Asn Asn Pro Leu Thr Phe Gln Gln Arg Pro Ser Val His
    450                 455                 460

Asn Ser Phe Gln Leu Gly Arg Arg Glu Thr Asp Phe Lys Asp Ala Ser
465                 470                 475                 480

Ser Gly Ala Gln Pro Phe Gly Asp Ser Leu Gly Phe Phe Met Gln Thr
                485                 490                 495

Thr Phe Asp Glu Ala Pro Asn Arg Leu Ser Ser Phe Lys Asn Gln Phe
            500                 505                 510

Gln Asp His Ser Tyr Ala Arg Gln Tyr Ala Asp Pro Tyr Leu Phe Met
        515                 520                 525

His Gln Gln Pro Ser Leu Thr Val Glu Ser Ser Arg Lys Met His Thr
    530                 535                 540

Glu Asn Asn Glu Met His Phe Trp Asn Gly Pro Ser Thr Ala Tyr Ser
545                 550                 555                 560

Asn Ser Arg Asp Gln Ala Gln Asp Phe Arg Phe Thr Glu His Pro Ser
                565                 570                 575

Asn Trp Leu Ser Pro Gln Phe Ser Arg Ala Glu Gln Pro Arg Val Ile
            580                 585                 590

Arg Pro His Ala Ser Ile Ala Pro Ile Glu Leu Glu Lys Thr Thr Glu
        595                 600                 605

Gly Ser Asp Phe Lys Ile Phe Gly Phe Lys Val Asp Thr Ala Ser Ala
    610                 615                 620

Gly Phe Asn His Leu Asn Ser Pro Met Ala Gly Thr His Glu Pro Val
625                 630                 635                 640

Leu Gln Thr Gln Pro Ser Val Ser Leu Asp His Leu Gln Thr Asp Cys
                645                 650                 655

Ser Pro Glu Val Ser Leu Ser Ile Ala Gly Thr Thr Glu Asn Glu Lys
            660                 665                 670

Asn Met Gln Gln Cys Pro Gln Ser Ser Lys Asp Val Gln Ser Lys Ser
        675                 680                 685

His Gly Ala Ser Thr Arg Ser Cys Thr Lys Val His Lys Gln Gly Val
    690                 695                 700

Ala Leu Gly Arg Ser Val Asp Leu Ser Lys Phe Val Asp Tyr Asp Glu
705                 710                 715                 720

Leu Thr Ala Glu Leu Asp Lys Met Phe Glu Phe Asp Gly Glu Leu Leu
                725                 730                 735

Ser Ser Asn Lys Asn Trp Gln Ile Val Tyr Thr Asp Asn Glu Gly Asp
            740                 745                 750

Met Met Leu Val Gly Asp Asp Pro Trp Glu Glu Phe Cys Ser Ile Val
        755                 760                 765

Arg Lys Ile Cys Ile Tyr Thr Lys Glu Glu Val Gln Lys Met Asn Ser
    770                 775                 780

Lys Pro Ser Gly Pro Lys Lys Glu Glu Gly Ser Val Asp Gly Asp Gly
785                 790                 795                 800

Ala Thr Glu Lys Ala His Leu Pro Ala Ser Ser Asp Leu Asn Ser Ser
                805                 810                 815

Gln Leu Gly Cys Val Lys Ala Asp
            820

<210>  10
<211>  805
<212>  PRT
<213>  Zea mays

<400>  10

Met Pro Pro Ala Thr Ala Met Ala Pro Pro Ser Gln Pro Pro Ser Asn
1               5                   10                  15

Ser Gly Asp Pro Leu Tyr Pro Glu Leu Trp Arg Ala Cys Ala Gly Pro
            20                  25                  30

Leu Val Thr Val Pro Arg Val Gly Asp Leu Val Phe Tyr Phe Pro Gln
        35                  40                  45

Gly His Ile Glu Gln Val Glu Ala Ser Met Asn Gln Val Ala Gly Asn
    50                  55                  60

Gln Met Arg Leu Tyr Asp Leu Pro Pro Lys Leu Leu Cys Arg Val Leu
65                  70                  75                  80

Asn Val Glu Leu Lys Ala Glu Thr Asp Thr Asp Glu Val Tyr Ala Gln
                85                  90                  95

Ile Met Leu Met Pro Glu Pro Glu Gln Thr Asp Val Pro Ala Glu Lys
            100                 105                 110

Pro Ser Ser Ala Pro Ala Ala Ser Pro Arg Pro Ala Val Arg Ser Phe
        115                 120                 125

Cys Lys Thr Leu Thr Ala Ser Asp Thr Ser Thr His Gly Gly Phe Ser
    130                 135                 140

Val Leu Arg Arg His Ala Asp Glu Cys Leu Pro Pro Leu Asp Met Thr
145                 150                 155                 160

Gln Ser Pro Pro Thr Gln Glu Leu Val Ala Lys Asp Leu His Gly Met
                165                 170                 175

Glu Trp Arg Phe Arg His Ile Phe Arg Gly Gln Pro Arg Arg His Leu
            180                 185                 190

Leu Gln Ser Gly Trp Ser Val Phe Val Ser Ser Lys Arg Leu Val Ala
        195                 200                 205

Gly Asp Ala Phe Ile Phe Leu Arg Gly Glu Asn Gly Glu Leu Arg Val
    210                 215                 220

Gly Val Arg Arg Ala Met Arg Gln Leu Ser Asn Val Pro Ser Ser Val
225                 230                 235                 240

Ile Ser Ser Gln Ser Met His Leu Gly Val Leu Ala Thr Ala Trp His
                245                 250                 255

Ala Ile Asn Thr Lys Ser Met Phe Thr Val Tyr Tyr Lys Pro Arg Thr
            260                 265                 270

Ser Pro Ser Glu Phe Ile Ile Pro Tyr Asp Gln Tyr Met Glu Ser Val
        275                 280                 285

Lys Asn Asn Tyr Ser Ile Gly Met Arg Phe Arg Met Arg Phe Glu Gly
    290                 295                 300

Glu Glu Ala Pro Glu Gln Arg Phe Thr Gly Thr Ile Val Gly Cys Glu
305                 310                 315                 320

Asn Leu Asp Pro Leu Trp Pro Asp Ser Ser Trp Arg Tyr Leu Lys Val
                325                 330                 335

Arg Trp Asp Glu Pro Ser Thr Ile Pro Arg Pro Asp Arg Val Ser Pro
            340                 345                 350

Trp Lys Ile Glu Pro Ala Ser Ser Pro Pro Val Asn Pro Leu Val His
        355                 360                 365

Ser Ser Arg Ala Lys Arg Pro Arg Gln Asn Val Pro Pro Pro Ser Leu
    370                 375                 380

Glu Ser Ser Val Leu Thr Lys Glu Gly Ala Thr Lys Val Glu Ile Asp
385                 390                 395                 400

Ser Ala Gln Thr Gln His Gln Asn Ser Val Leu Gln Gly Gln Glu Gln
                405                 410                 415

Met Thr Leu Arg Asn Asn Leu Thr Glu Ser Ala Asp Ser Asp Ser Thr
            420                 425                 430

Val Gln Lys Pro Met Met Trp Ser Pro Ser Leu Asn Gly Lys Ala His
        435                 440                 445

Thr His Phe Gln Gln Arg Pro Ala Met Asp Asn Trp Met Pro Leu Gly
    450                 455                 460

Arg Arg Glu Asn Asp Phe Lys Asp Thr Arg Ser Ala Phe Lys Asp Ala
465                 470                 475                 480

Arg Thr Ala Ser Gln Ser Phe Gly Asp Thr Gln Gly Phe Phe Val Gln
                485                 490                 495

Ala Tyr Asp Asp Asn His His Arg Leu Ser Phe Lys Asn Gln Gly Ser
            500                 505                 510

Thr His Arg Phe Ala Asp Pro Tyr Phe Tyr Met Pro Gln Gln Ser Ser
        515                 520                 525

Val Thr Val Glu Ser Ser Thr Arg Thr Gln Thr Ala Asn Asn Asp Leu
    530                 535                 540

Cys Phe Trp Gly Asp Gln Asn Ala Met Tyr Gly Asn Pro Ser Asp Gln
545                 550                 555                 560

Gln Gln Gly Phe Asn Phe Gly Gln Asn Pro Ser Ser Trp Leu Asn Gln
                565                 570                 575

Pro Phe Pro Gln Val Glu Gln Pro Arg Val Val Arg Pro His Ala Thr
            580                 585                 590

Val Ala Pro Phe Asp Leu Glu Lys Thr Arg Glu Gly Ser Gly Phe Lys
        595                 600                 605

Ile Phe Gly Phe Gln Val Asp Thr Thr Ser Pro Ser Pro Val Gln Leu
    610                 615                 620

Ser Ser Pro Leu Pro Ala Ile Gln Glu His Val Leu Gln Thr Arg Pro
625                 630                 635                 640

Ser Ala Pro Val Asn Glu Leu Gln Pro Val Gln Ile Glu Cys Leu Pro
                645                 650                 655

Glu Gly Ser Val Ser Thr Ala Gly Thr Ala Thr Glu Asn Ile Gln Gln
            660                 665                 670

Ala Pro Gln Ser Ser Lys Asp Ile Gln Ser Lys Ser Gln Gly Ala Ser
        675                 680                 685

Thr Arg Ser Cys Thr Lys Val His Lys Gln Gly Val Ala Leu Gly Arg
    690                 695                 700

Ser Val Asp Leu Ser Lys Phe Thr Asp Tyr Gly Glu Leu Gln Ala Glu
705                 710                 715                 720

Leu Asp Lys Met Phe Asp Phe Glu Gly Glu Leu Val Ser Gly Ser Gln
                725                 730                 735

Asn Trp Gln Ile Val Tyr Thr Asp Asp Glu Gly Asp Met Met Leu Val
            740                 745                 750

Gly Asp Asp Pro Trp Glu Glu Phe Cys Ser Ile Val Arg Lys Ile Tyr
        755                 760                 765

Ile Tyr Thr Lys Glu Glu Val Gln Lys Met Asn Ser Lys Ser Ala Ala
    770                 775                 780

Pro Arg Lys Glu Glu Ser Pro Ala Ala Gly Gly Gly Gly Gly Cys Ala

785                 790                 795                 800

Ala Ala Thr Asn Glu
                805

<210>  11
<211>  1984
<212>  DNA
<213>  Zea mays

<400>  11
gagtccttgc gactgcatgg cacgccatta acacgaagtc catgttcact gtctactaca      60

agcctaggac gagcccttca gagttcatca taccgtacga tcaatatatg gagtcggtaa     120

aaaataatta ttctattggc atgagattca ggatgaggtt tgaaggagaa gaggcgccag     180

agcagaggtt tactggtaca atagttggtt gtgaaaatct tgacccacta tggcctgatt     240

ccagttggag atacttgaag gtgcgttggg atgagccttc tactatcccg cggccagata     300

gggtctctcc ttggaagata gagcctgctt catcgcctcc tgttaatcca ctcgtgcatt     360

cttctagagc aaaaagacct agacaaaacg ttcctccacc ttcacttgaa tcatctgttc     420

tcacaaaaga aggtgcaacc aaggtcgaaa tcgattcagc tcaaacacaa caccaaaatt     480

cggtcttgca aggtcaggag cagatgacct tgaggaacaa cctgactgaa agcgctgatt     540

ctgattccac tgttcagaag ccgatgatgt ggtccccatc actcaatggg aaagcccaca     600

ctcattttca gcagagacct gctatggata attggatgcc attgggaagg cgtgaaaatg     660

acttcaagga cacccgttct gccttcaagg atgcccgcac cgcctctcaa tcatttggag     720

atacacaggg tttttttgtg caagcttatg atgacaatca tcaccgtctc tctttcaaaa     780

atcagggttc aactcatcgc ttcgcagatc cgtacttcta tatgcctcaa caatcgtcag     840

tgacggttga atcaagcaca aggacacaaa cagcaaacaa tgacttgtgt ttctggggtg     900

accagaatgc tatgtacggc aatccaagtg accaacaaca gggtttcaac tttggacaaa     960

acccatcaag ttggttgaac cagccatttc cccaggttga acagccacga gtggtcaggc    1020

ctcatgcaac ggttgctcca tttgatctgg agaagactag agaaggaagt ggcttcaaga    1080

tttttgggtt ccaagttgat acaaccagtc catctcctgt ccaattgagc tctccattgc    1140

ccgctatcca ggagcatgtg ttacaaactc gaccatcggc accggtgaat gaattgcaac    1200

ctgtgcaaat tgagtgcttg cctgagggat ctgtaagcac agctggaact gcaacggaga    1260

acattcagca agccccgcag agttcaaaag atatccaaag caagtcccag ggtgcttcaa    1320

caaggagctg tacaaaggtt cataagcaag gagtcgcact tggcaggtcg gtggacctct    1380

ctaaattcac cgactacggc gaactccaag cagagctgga caagatgttt gacttcgagg    1440

gtgaattggt atctggcagc caaaactggc agatcgtcta taccgacgac gagggcgata    1500

tgatgcttgt gggagacgac ccgtgggaag agttctgcag catagtgcgc aagatctaca    1560

tctacacgaa ggaggaggtc cagaagatga actcgaaatc agctgcgcca aggaaggagg 1620

aatctccggc agcgggcggc ggaggaggtt gcgccgccgc cacaaacgag tagtatggtg 1680

gtttgctctt ggtggcaagt tgaggccaag gttttactac tgctattgtt attgctgatt 1740

tgctctcccc gccttccggt gtaaatatct gtgtgtaccc agtcccggcc ttgtgctagt 1800

aagtagcaag cagtgttacc ttttggtatt ttaaccagca gctaagttcg cggcatttta 1860

gtccctgtaa gcggtaagca gctctgtagc gtgagataga tggccaagcg ccggtacgta 1920

cgtgtagtgt ctggtcttct cactgagtgt actataaaga gttaatatag gagctgaccc 1980

tgtg 1984

<210> 12
<211> 792
<212> DNA
<213> Zea mays

<400> 12
cacatcaaaa ttcggtcttg caaagtcaag agcagatgag ctttaggaac aacctgactg 60

aaagcaccga ctctgattcc actgttcaga agcagatgat gtggtcccca tcacccaatg 120

cgaaagttca cactaatttt cagccgagac ctgctatgga taactggatg ccattgggaa 180

ggcgtgaaac tgacttcaag gacacccgtt ctgccttcaa ggatgcccgc accgcttctc 240

aatcatttgg agatacacag gggttcttta tgcaagctta tgacgacagt cgtcaccgtc 300

tttctttcaa caatcagttt caagatcagg gttcagctca tcgctttgca gatccatact 360

tctatatgcc tcaacaacct tctctgacgg ttgaatcggg cacaaggacg caaacagcga 420

acaatgactt gcgtttttgg agtgagcgga attcgatgta tgggaatcca agtgaccaac 480

agcagggttt cagctttggg caaaacccat caagttggtt gaaccagcca catccccagg 540

ttgaacagtc acgagtggtc aggccgcatg caacggttgc tccatttgat ttggagaaga 600

ctagagaagg cagtgagttc aagatttttg gtttccaagt tgatacaacc aatccatcac 660

ctgtccaatt gagctctcca ttgtctgcta tccgagagca tgtggtacaa actcgaccat 720

cggcaccagt gaatgaatcg caacctgtgc aaatcgagtg cttgcctgag gtgtccgtaa 780

gcacagctgg aa 792

<210> 13
<211> 215
<212> DNA
<213> Triticum aestivum

<400> 13
ttccgtgggc aacctaggag gcatctcctt cagagcggtt ggagtgtgtt tgtcagttcc 60

aaaaggcttg tagctgggga tgccttcatt tttctcagag gagagagtgg tgagcttcgt 120

ggtgttaggc gggctatgag acagctgtcc aacgtgcctt cttcagtcat atctagtcat 180

agcatgcacc ttggagtcct tgcaactgca tggca 215

<210>  14
<211>  250
<212>  DNA
<213>  Oryza sativa

<400>  14
ccgcgtctga caccagcacg cacggcggct tctctgtact ccgccgccac gctgatgagt      60

gcctccctcc cctggatatg acccagtcgc cacccacaca ggagctcgtg gccaaggatc     120

tgcacagcat ggactggcgc ttccgtcaca tctttcgcgg gcaaccgagg aggcatctcc     180

ttcagagtgg ttggagtgtg tttgtcagtt ccaaaaggct tgttgctggg gatgctttca     240

ttttcctcag                                                            250


<210>  15
<211>  250
<212>  DNA
<213>  Sorghum bicolor

<400>  15
gacctcgtct tctactttcc ccagggacac atcgagcagg tggaggcgtc catgaaccag      60

gtcgccggga accagatgcg cctctacgat ctgccctcca agctgctctg ccgcgtgctc     120

aacgtcgagc ttaaggcgga gacggacacc gacgaggtct acgcgcagat catgctcatg     180

ccagagcccg agcaaaccga tgtggcggct gagaaggcga gttctgcgtc cgctgcgtcg     240

ccgaggccag                                                            250


<210>  16
<211>  250
<212>  DNA
<213>  Brachypodium distachyon

<400>  16
acctcgtctt ctacttcccg cagggccaca tcgagcaggt ggaggcctcc atgaaccagg      60

tcgccgacaa ccagatgcgc ctctacgatc tgccttccaa gctgctctgc agcgtcatca     120

acgtcgagct caaggcggag gccgacaccg acgaggtcta cgcgcaagtc atgctcatcc     180

cggagaacga tcaaaacgag atggcggtgg agaagtcgag ctccaaagca gccaccacgc     240

tagcaaagcc                                                            250


<210>  17
<211>  502
<212>  DNA
<213>  Artificial

<220>
<223>  CSVMV promoter

<400>  17
aaggtaatta tccaagatgt agcatcaaga atccaatgtt tacgggaaaa actatggaag      60

tattatgtga gctcagcaag aagcagatca atatgcggca catatgcaac ctatgttcaa     120

aaatgaagaa tgtacagata caagatccta tactgccaga atacgaagaa gaatacgtag 180

aaattgaaaa agaagaacca ggcgaagaaa agaatcttga agacgtaagc actgacgaca 240

acaatgaaaa gaagaagata aggtcggtga ttgtgaaaga gacatagagg acacatgtaa 300

ggtggaaaat gtaagggcgg aaagtaacct tatcacaaag gaatcttatc ccccactact 360

tatcctttta tatttttccg tgtcattttt gcccttgagt tttcctatat aaggaaccaa 420

gttcggcatt tgtgaaaaca agaaaaaatt tggtgtaagc tattttcttt gaagtactga 480

ggatacaact tcagagaaat tt 502

<210> 18
<211> 4432
<212> DNA
<213> Artificial

<220>
<223> RNAi construct for maize

<400> 18
tcgagaaggt aattatccaa gatgtagcat caagaatcca atgtttacgg gaaaaactat 60

ggaagtatta tgtgagctca gcaagaagca gatcaatatg cggcacatat gcaacctatg 120

ttcaaaaatg aagaatgtac agatacaaga tcctatactg ccagaatacg aagaagaata 180

cgtagaaatt gaaaaagaag aaccaggcga agaaaagaat cttgaagacg taagcactga 240

cgacaacaat gaaaagaaga agataaggtc ggtgattgtg aaagagacat agaggacaca 300

tgtaaggtgg aaaatgtaag ggcggaaagt aaccttatca caaaggaatc ttatccccca 360

ctacttatcc ttttatattt ttccgtgtca tttttgccct tgagttttcc tatataagga 420

accaagttcg gcatttgtga aaacaagaaa aaatttggtg taagctattt tctttgaagt 480

actgaggata caacttcaga gaaatttgtc cctcccccct ccccctccgc cgccgccgcg 540

ccggtaacca ccccgcccct ctcctctttc tttctccgtt ttttttttcc gtctcggtct 600

cgatctttgg ccttggtagt ttgggtgggc gagaggcggc ttcgtgcgcg cccagatcgg 660

tgcgcgggag gggcgggatc tcgcggctgg ggctctcgcc ggcgtggatc cggcccggat 720

ctcgcgggga atggggctct cggatgtaga tctgcgatcc gccgttgttg ggggagatga 780

tggggggttt aaaatttccg ccatgctaaa caagatcagg aagaggggaa aagggcacta 840

tggtttatat ttttatatat ttctgctgct tcgtcaggct tagatgtgct agatctttct 900

ttcttctttt tgtgggtaga atttgaatcc ctcagcattg ttcatcggta gtttttcttt 960

tcatgatttg tgacaaatgc agcctcgtgc ggagcttttt tgtaggtaga cgataagctt 1020

gatctccacc ctagaactag tggatccccc gggctgcagg aattcgatat caccactttg 1080

tacaagaaag ctgggtctag atatctcgag tgcggccgcg ggaattcgat tcacatcaaa 1140

attcggtctt gcaaagtcaa gagcagatga gctttaggaa caacctgact gaaagcaccg 1200

actctgattc cactgttcag aagcagatga tgtggtcccc atcacccaat gcgaaagttc 1260

acactaattt tcagccgaga cctgctatgg ataactggat gccattggga aggcgtgaaa 1320

ctgacttcaa ggacacccgt tctgccttca aggatgcccg caccgcttct caatcatttg 1380

gagatacaca ggggttcttt atgcaagctt atgacgacag tcgtcaccgt ctttctttca 1440

acaatcagtt tcaagatcag ggttcagctc atcgctttgc agatccatac ttctatatgc 1500

ctcaacaacc ttctctgacg gttgaatcgg gcacaaggac gcaaacagcg aacaatgact 1560

tgcgtttttg gagtgagcgg aattcgatgt atgggaatcc aagtgaccaa cagcagggtt 1620

tcagctttgg gcaaaaccca tcaagttggt tgaaccagcc acatccccag gttgaacagt 1680

cacgagtggt caggccgcat gcaacggttg ctccatttga tttggagaag actagagaag 1740

gcagtgagtt caagattttt ggtttccaag ttgatacaac caatccatca cctgtccaat 1800

tgagctctcc attgtctgct atccgagagc atgtggtaca aactcgacca tcggcaccag 1860

tgaatgaatc gcaacctgtg caaatcgagt gcttgcctga ggtgtccgta agcacagctg 1920

gaaaatcact agggttcctt taaagcctgc ttttttgtac aaacttgtga tatcaagctt 1980

cgtggatcca gatggccgcg gcatccaggt aaaacaaaca aaaacggatc tgatgcttcc 2040

attcctccgt ttctcgtagt agcgcgcttc gatctgtggg tggatctggg tgatcctggg 2100

gtgtggttcg ttctgtttga tagatctgtc ggtggatctg gccttctgtg gttgtcgatg 2160

tccggatctg cgttttgatc agtggtagtt cgtggatctg gcgaaatgtt ttggatctgg 2220

cagtgagacg ctaagaatcg ggaaatgatg caatattagg ggggtttcgg atggggatcc 2280

actgaattag tctgtctccc tgctgataat ctgttccttt ttggtagatc tggttagtgt 2340

atgtttgttt cggatagatc tgatcaatgc ttgtttgttt tttcaaattt tctacctagg 2400

gtgtatagga atggcatgcg gatctggttg gattgccatg atccgtgctg aaatgcccct 2460

ttggttgatg gatcttgata ttttactgct gttcacctag atttgtactc ccgtttatac 2520

ttaatttgtt gcttattatg aatagatctg taacttaggc acatgtatgg acggagtatg 2580

tggatctgta gtatgtacat tgctgcgagc taagaactat ttcagagcaa gcacagaaaa 2640

aaatatttag acagattggg caactatttg atggtctttg gtatcatgct ttgtagtgct 2700

cgtttctgcg tagtaatctt ttgatctgat ctgaagatag gtgctattat attcttaaag 2760

gtcattagaa cgctatctga aaggctgtat tatgtggatt ggttcacctg tgactccctg 2820

ttcgtcttgt cttgataaat cctgtgataa aaaaaattct taaggcgtaa tttgttgaaa 2880

tcttgttttg tcctatgcag cctgatccat cctgcaggaa ttcctcgatc gaggtcgacg 2940

gtatcgataa gcttgatatc acaagtttgt acaaaaaagc aggctttaaa ggaaccctag 3000

tgattttcca gctgtgctta cggacacctc aggcaagcac tcgatttgca caggttgcga 3060

ttcattcact ggtgccgatg gtcgagtttg taccacatgc tctcggatag cagacaatgg 3120

agagctcaat tggacaggtg atggattggt tgtatcaact tggaaaccaa aaatcttgaa 3180

ctcactgcct tctctagtct tctccaaatc aaatggagca accgttgcat gcggcctgac 3240

cactcgtgac tgttcaacct ggggatgtgg ctggttcaac caacttgatg ggttttgccc 3300

aaagctgaaa ccctgctgtt ggtcacttgg attcccatac atcgaattcc gctcactcca 3360

aaaacgcaag tcattgttcg ctgtttgcgt ccttgtgccc gattcaaccg tcagagaagg 3420

ttgttgaggc atatagaagt atggatctgc aaagcgatga gctgaaccct gatcttgaaa 3480

ctgattgttg aaagaaagac ggtgacgact gtcgtcataa gcttgcataa agaacccctg 3540

tgtatctcca aatgattgag aagcggtgcg ggcatccttg aaggcagaac gggtgtcctt 3600

gaagtcagtt tcacgccttc ccaatggcat ccagttatcc atagcaggtc tcggctgaaa 3660

attagtgtga actttcgcat tgggtgatgg ggaccacatc atctgcttct gaacagtgga 3720

atcagagtcg gtgctttcag tcaggttgtt cctaaagctc atctgctctt gactttgcaa 3780

gaccgaattt tgatgtgaat cgaattcccg cggccgcact cgagatatct agacccagct 3840

ttcttgtaca aagtggtgat atcgaattcc tgcagcccgg gggatccact agttctagag 3900

cggccgccac cgcggtggag atcctaaatg ctcttaactg agctaattat gtaatgcaca 3960

tacacatatt tacatagata tgcatattta tatatagcat gtatattgta ctacatgcat 4020

tgcttcttaa tacatgtagt aaagatatat gcaaaaatag tcgaaagatt tgtttacata 4080

taaaatcacc aatatttatt gttattgtat tttcatgaat aaagtaataa gattatttgt 4140

ctaatatttt gatttactag tactagaaat gaaaaggaat atgcacaatt tcagcattat 4200

agtttggtag gcaaaatgga gtgagaatag agtttcatag tatatactaa ggttcttaat 4260

tgtgcaaata gttgatacaa gtcacatggg ccaagtttgt aaatcttaaa tcgaaatatg 4320

ccttcttctt tttttgcatg aaaatgctag taatttataa gtgtgttttt caataagaga 4380

tgctaaatac caaaattaac ctagttttca gtgagcgctt gcattattgt gg 4432

<210> 19
<211> 24
<212> DNA
<213> Artificial

<220>
<223> primer

<400> 19
cacatcaaaa ttcggtcttg caaa 24

<210> 20
<211> 21
<212> DNA
<213> Artificial

<220>
<223> primer

<400> 20

ttccagctgt gcttacggac a                                                21

<210>  21
<211>  20
<212>  DNA
<213>  Artificial

<220>
<223>  primer

<400>  21
agccttctac tatcccgcgg                                                  20

<210>  22
<211>  24
<212>  DNA
<213>  Artificial

<220>
<223>  primer

<400>  22
aggctctatc ttccagggag agac                                             24

<210>  23
<211>  22
<212>  DNA
<213>  Artificial

<220>
<223>  primer

<400>  23
tggtgaagat gataccgacc aa                                               22

<210>  24
<211>  20
<212>  DNA
<213>  Artificial

<220>
<223>  primer

<400>  24
agcaaccgtc tgcctcatgt                                                  20

<210>  25
<211>  20
<212>  DNA
<213>  Artificial

<220>
<223>  primer

<400>  25
tgctggacct cgattggttt                                                  20

<210>  26
<211>  19

<212>  DNA
<213>  Artificial

<220>
<223>  primer

<400>  26
gtcctgcatc aaccgcttg                                                    19


<210>  27
<211>  826
<212>  PRT
<213>  Hordeum vulgare

<400>  27

Met Pro Pro Ala Ala Met Pro Pro Pro Pro Pro Pro Pro Gln Gly Thr
1               5                   10                  15

Ser Thr Gly Asp Pro Leu Tyr Asp Glu Leu Trp His Ala Cys Ala Gly
            20                  25                  30

Pro Leu Val Thr Val Pro Arg Val Gly Asp Met Val Tyr Tyr Phe Pro
        35                  40                  45

Gln Gly His Ile Glu Gln Val Glu Ala Ser Met Asn Gln Val Ala Ala
    50                  55                  60

Asn Gln Met Arg Leu Tyr Asp Leu Pro Ser Lys Leu Leu Cys Arg Val
65                  70                  75                  80

Leu Asn Val Glu Leu Lys Ala Glu Ala Asp Thr Asp Glu Val Tyr Ala
                85                  90                  95

Gln Val Met Leu Met Pro Glu Pro Glu Gln Ser Glu Ala Ala Ala Thr
            100                 105                 110

Thr Thr Glu Lys Ser Ser Ser Ala Thr Gly Gly Thr Met Pro Ala Arg
        115                 120                 125

Pro Ala Val Arg Ser Phe Cys Lys Thr Leu Thr Ala Ser Asp Thr Ser
    130                 135                 140

Thr His Gly Gly Phe Ser Val Leu Arg Arg His Ala Asp Glu Cys Leu
145                 150                 155                 160

Pro Pro Leu Asp Met Thr Gln Ser Pro Pro Thr Gln Glu Leu Val Ala
                165                 170                 175

Lys Asp Leu His Gly Met Glu Trp Arg Phe Arg His Ile Phe Arg Gly
            180                 185                 190

Gln Pro Arg Arg His Leu Leu Gln Ser Gly Trp Ser Val Phe Val Ser
        195                 200                 205

Ser Lys Arg Leu Val Ala Gly Asp Ala Phe Ile Phe Leu Arg Gly Glu
    210                 215                 220

Ser Gly Glu Leu Arg Val Gly Val Arg Arg Ala Met Arg Gln Leu Ser
225                 230                 235                 240

Asn Ile Ala Ser Ser Val Ile Ser Ser His Ser Met His Leu Gly Val
                245                 250                 255

Leu Ala Thr Ala Trp His Ala Ile Asn Thr Lys Thr Met Phe Thr Val
            260                 265                 270

Tyr Tyr Lys Pro Arg Thr Ser Arg Ser Glu Phe Ile Ile Pro Tyr Asp
        275                 280                 285

Lys Tyr Thr Glu Ser Val Lys Asn Ile Tyr Ser Ile Gly Thr Arg Phe
    290                 295                 300

Lys Met Arg Phe Glu Gly Glu Glu Ala Pro Glu Gln Arg Phe Thr Gly
305                 310                 315                 320

Thr Ile Val Gly Ser Asp Asn Leu Asp Gln Leu Trp Pro Glu Ser Ser
                325                 330                 335

Trp Arg Ser Leu Lys Val Arg Trp Asp Glu Ser Ser Thr Ile Pro Arg
            340                 345                 350

Pro Asp Arg Val Ser Pro Trp Glu Ile Glu Pro Ala Ser Ser Pro Pro
        355                 360                 365

Val Asn Pro Leu Pro Leu Ser Arg Ala Lys Arg Ser Arg Pro Asn Val
    370                 375                 380

Pro Pro Ala Ser Pro Glu Ser Ser Val Arg Thr Lys Glu Gly Ala Thr
385                 390                 395                 400

Lys Ala Asp Met Asp Cys Ala Gln Ala Gln Arg Asn Gln Asn Asn Thr
                405                 410                 415

Val Leu Pro Gly His Glu Gln Arg Ser Asn Lys Leu Thr Asp Ile Asn
            420                 425                 430

Asp Phe Asp Ala Thr Val Gln Lys Pro Met Val Trp Ser Thr Pro Pro
        435                 440                 445

Pro Asn Ile Gly Lys Asn Asn Pro Leu Thr Phe Gln Gln Arg Pro Ser

450                 455                 460

Val His Asn Ser Ile Gln Leu Arg Arg Arg Glu Ala Asp Phe Lys Asp
465                 470                 475                 480

Ala Ser Ser Gly Ala Gln His Phe Gly Asp Ser Leu Gly Phe Phe Met
                485                 490                 495

Gln Thr Thr Phe Asp Glu Ala Pro Asn Arg Leu Gly Ser Phe Lys Asn
            500                 505                 510

Gln Phe Gln Asp His Ser Tyr Ala Arg Gln Phe Ala Asp Pro Tyr Leu
        515                 520                 525

Phe Met His Gln Gln Pro Ser Leu Thr Val Glu Ser Ser Arg Lys Met
    530                 535                 540

His Thr Glu Asn Asn Glu Leu His Phe Trp Asn Gly Pro Gly Thr Val
545                 550                 555                 560

Tyr Gly Asn Ser Ile Asp His Val Gln Asp Phe Arg Phe Lys Glu His
                565                 570                 575

Pro Ser Asn Trp Leu Ser Pro Gln Phe Ser Arg Ala Glu Gln Pro Arg
            580                 585                 590

Val Ile Arg Pro His Ala Ser Ile Ala Pro Ile Glu Leu Glu Lys Thr
        595                 600                 605

Thr Glu Gly Ser Asp Phe Lys Ile Phe Gly Phe Lys Val Asp Thr Ala
    610                 615                 620

Ser Ala Gly Phe Asn His Leu Asn Ser Pro Met Ala Ala Thr His Glu
625                 630                 635                 640

Pro Val Leu Gln Thr Gln Pro Ser Val Ser Leu Asp His Leu Gln Thr
                645                 650                 655

Asp Cys Ser Pro Glu Val Ser Leu Ser Ile Ala Gly Thr Thr Asp Asn
            660                 665                 670

Glu Lys Asn Ile Gln Gln Cys Pro Gln Ser Ser Lys Asp Val Gln Ser
        675                 680                 685

Lys Ser His Gly Ala Ser Thr Arg Ser Cys Thr Lys Val His Lys Gln
    690                 695                 700

Gly Val Ala Leu Gly Arg Ser Val Asp Leu Ser Lys Phe Val Asp Tyr
705                 710                 715                 720

Asp Glu Leu Thr Ala Glu Leu Asp Lys Met Phe Asp Phe Asp Gly Glu
                725                 730                 735

Leu Met Ser Ser Asn Lys Asn Trp Gln Ile Val Tyr Thr Asp Asn Glu
            740                 745                 750

Gly Asp Met Met Leu Val Gly Asp Asp Pro Trp Glu Glu Phe Cys Ser
        755                 760                 765

Met Val Arg Lys Ile Cys Ile Tyr Thr Lys Glu Glu Val Gln Lys Met
    770                 775                 780

Asn Ser Lys Pro Ser Asp Ala Arg Lys Glu Glu Gly Ser Val Glu Gly
785                 790                 795                 800

Asp Gly Ala Thr Glu Lys Val His Leu Pro Val Ser Ser Asp Leu Asn
                805                 810                 815

Ser Ser Gln Leu Gly Cys Val Lys Ala Asp
            820                 825

<210>  28
<211>  859
<212>  PRT
<213>  Arabidopsis thaliana

<400>  28

Met Ala Ser Ser Glu Val Ser Met Lys Gly Asn Arg Gly Gly Asp Asn
1               5                   10                  15

Phe Ser Ser Ser Gly Phe Ser Asp Pro Lys Glu Thr Arg Asn Val Ser
            20                  25                  30

Val Ala Gly Glu Gly Gln Lys Ser Asn Ser Thr Arg Ser Ala Ala Ala
        35                  40                  45

Glu Arg Ala Leu Asp Pro Glu Ala Ala Leu Tyr Arg Glu Leu Trp His
    50                  55                  60

Ala Cys Ala Gly Pro Leu Val Thr Val Pro Arg Gln Asp Asp Arg Val
65                  70                  75                  80

Phe Tyr Phe Pro Gln Gly His Ile Glu Gln Val Glu Ala Ser Thr Asn
                85                  90                  95

Gln Ala Ala Glu Gln Gln Met Pro Leu Tyr Asp Leu Pro Ser Lys Leu
            100                 105                 110

Leu Cys Arg Val Ile Asn Val Asp Leu Lys Ala Glu Ala Asp Thr Asp
        115                 120                 125

Glu Val Tyr Ala Gln Ile Thr Leu Leu Pro Glu Ala Asn Gln Asp Glu
    130                 135                 140

Asn Ala Ile Glu Lys Glu Ala Pro Leu Pro Pro Pro Pro Arg Phe Gln
145                 150                 155                 160

Val His Ser Phe Cys Lys Thr Leu Thr Ala Ser Asp Thr Ser Thr His
                165                 170                 175

Gly Gly Phe Ser Val Leu Arg Arg His Ala Asp Glu Cys Leu Pro Pro
            180                 185                 190

Leu Asp Met Ser Arg Gln Pro Pro Thr Gln Glu Leu Val Ala Lys Asp
        195                 200                 205

Leu His Ala Asn Glu Trp Arg Phe Arg His Ile Phe Arg Gly Gln Pro
    210                 215                 220

Arg Arg His Leu Leu Gln Ser Gly Trp Ser Val Phe Val Ser Ser Lys
225                 230                 235                 240

Arg Leu Val Ala Gly Asp Ala Phe Ile Phe Leu Arg Gly Glu Asn Gly
                245                 250                 255

Glu Leu Arg Val Gly Val Arg Arg Ala Met Arg Gln Gln Gly Asn Val
            260                 265                 270

Pro Ser Ser Val Ile Ser Ser His Ser Met His Leu Gly Val Leu Ala
        275                 280                 285

Thr Ala Trp His Ala Ile Ser Thr Gly Thr Met Phe Thr Val Tyr Tyr
    290                 295                 300

Lys Pro Arg Thr Ser Pro Ser Glu Phe Ile Val Pro Phe Asp Gln Tyr
305                 310                 315                 320

Met Glu Ser Val Lys Asn Asn Tyr Ser Ile Gly Met Arg Phe Lys Met
                325                 330                 335

Arg Phe Glu Gly Glu Glu Ala Pro Glu Gln Arg Phe Thr Gly Thr Ile
            340                 345                 350

Val Gly Ile Glu Glu Ser Asp Pro Thr Arg Trp Pro Lys Ser Lys Trp
        355                 360                 365

Arg Ser Leu Lys Val Arg Trp Asp Glu Thr Ser Ser Ile Pro Arg Pro

370                 375                 380

Asp Arg Val Ser Pro Trp Lys Val Glu Pro Ala Leu Ala Pro Pro Ala
385                 390                 395                 400

Leu Ser Pro Val Pro Met Pro Arg Pro Lys Arg Pro Arg Ser Asn Ile
                405                 410                 415

Ala Pro Ser Ser Pro Asp Ser Ser Met Leu Thr Arg Glu Gly Thr Thr
            420                 425                 430

Lys Ala Asn Met Asp Pro Leu Pro Ala Ser Gly Leu Ser Arg Val Leu
        435                 440                 445

Gln Gly Gln Glu Tyr Ser Thr Leu Arg Thr Lys His Thr Glu Ser Val
    450                 455                 460

Glu Cys Asp Ala Pro Glu Asn Ser Val Val Trp Gln Ser Ser Ala Asp
465                 470                 475                 480

Asp Asp Lys Val Asp Val Val Ser Gly Ser Arg Arg Tyr Gly Ser Glu
                485                 490                 495

Asn Trp Met Ser Ser Ala Arg His Glu Pro Thr Tyr Thr Asp Leu Leu
            500                 505                 510

Ser Gly Phe Gly Thr Asn Ile Asp Pro Ser His Gly Gln Arg Ile Pro
        515                 520                 525

Phe Tyr Asp His Ser Ser Ser Pro Ser Met Pro Ala Lys Arg Ile Leu
    530                 535                 540

Ser Asp Ser Glu Gly Lys Phe Asp Tyr Leu Ala Asn Gln Trp Gln Met
545                 550                 555                 560

Ile His Ser Gly Leu Ser Leu Lys Leu His Glu Ser Pro Lys Val Pro
                565                 570                 575

Ala Ala Thr Asp Ala Ser Leu Gln Gly Arg Cys Asn Val Lys Tyr Ser
            580                 585                 590

Glu Tyr Pro Val Leu Asn Gly Leu Ser Thr Glu Asn Ala Gly Gly Asn
        595                 600                 605

Trp Pro Ile Arg Pro Arg Ala Leu Asn Tyr Tyr Glu Glu Val Val Asn
    610                 615                 620

Ala Gln Ala Gln Ala Gln Ala Arg Glu Gln Val Thr Lys Gln Pro Phe
625                 630                 635                 640

Thr Ile Gln Glu Glu Thr Ala Lys Ser Arg Glu Gly Asn Cys Arg Leu
                645                 650                 655

Phe Gly Ile Pro Leu Thr Asn Asn Met Asn Gly Thr Asp Ser Thr Met
            660                 665                 670

Ser Gln Arg Asn Asn Leu Asn Asp Ala Ala Gly Leu Thr Gln Ile Ala
        675                 680                 685

Ser Pro Lys Val Gln Asp Leu Ser Asp Gln Ser Lys Gly Ser Lys Ser
    690                 695                 700

Thr Asn Asp His Arg Glu Gln Gly Arg Pro Phe Gln Thr Asn Asn Pro
705                 710                 715                 720

His Pro Lys Asp Ala Gln Thr Lys Thr Asn Ser Ser Arg Ser Cys Thr
                725                 730                 735

Lys Val His Lys Gln Gly Ile Ala Leu Gly Arg Ser Val Asp Leu Ser
            740                 745                 750

Lys Phe Gln Asn Tyr Glu Glu Leu Val Ala Glu Leu Asp Arg Leu Phe
        755                 760                 765

Glu Phe Asn Gly Glu Leu Met Ala Pro Lys Lys Asp Trp Leu Ile Val
    770                 775                 780

Tyr Thr Asp Glu Glu Asn Asp Met Met Leu Val Gly Asp Asp Pro Trp
785                 790                 795                 800

Gln Glu Phe Cys Cys Met Val Arg Lys Ile Phe Ile Tyr Thr Lys Glu
                805                 810                 815

Glu Val Arg Lys Met Asn Pro Gly Thr Leu Ser Cys Arg Ser Glu Glu
            820                 825                 830

Glu Ala Val Val Gly Glu Gly Ser Asp Ala Lys Asp Ala Lys Ser Ala
        835                 840                 845

Ser Asn Pro Ser Leu Ser Ser Ala Gly Asn Ser
    850                 855

## Description

The invention relates to the field of plant improvement, in particular of the improvement of yield for cereal plants.

Cereals are grasses (members of the monocot family Poaceae, also known as *Gramineae*) cultivated for the edible components of their grain, composed of the endosperm, germ and bran.

In the context of the present invention a cereal shall mean in particular maize, rice, wheat, barley, sorghum, millet, oats, rye, triticale (hybrid of wheat and rye), fonio, as well as two pseudocereals, namely buckwheat and quinoa.

In agriculture, yield is the amount of product harvested from a given acreage (eg weight of seeds per unit area). It is often expressed in metric quintals (1 q = 100 kg) per hectare in the case of cereals. It is becoming increasingly important to improve the yield of seed crops to feed an expanding population and, more recently, for biofuel production. One strategy to increase the yield is to increase the seed size, provided that there is not a concomitant decrease in seed number.

Some authors have described plants which present larger grains than control plants.

In particular, WO 2005/085453 deals, in its introduction, with the problem of increasing yield through increase of the seed size.

This patent application reviews different strategies in order to increase seed size, and cites some patent applications related to genes that are supposed to be of interest for increasing seed size. This patent application describes a mutant obtained in *Arabidopsis thaliana,* which presents larger seeds than controls.

This application speculates on the possibility to inhibit the gene *mnt* (as described), which is actually the *arf2* gene (for auxin response factor 2) in various plants in order to increase seed size.

Nevertheless, it is to be noted that this application does not provide experimental data on plants others than *A. thaliana* (some incomplete data is provided for *Brassica napus*) and that it specifies that the mature seeds of monocots and in particular of cereals, have a different structure than the *Arabidopsis* seeds (although there may be some similar steps during the seed development between rice and *Arabidopsis*).

Furthermore, the application only describes the *Arabidopsis* and B. *napus* sequences and only provides information on a rice partial DNA sequence.

The examples describe that the *mnt* mutant plants are self-sterile late in development, due to floral abnormalities (page 25, lines 1-2). The examples also indicate that the RNAi constructs made in *Arabidopsis* lead to plants that present the same phenotype than the mutants (page 39, paragraph 4c). It is also interesting to note that overexpression of *mnt* gene in *Arabidopsis* leads to plants presenting the same phenotype as the mutant (closed flowers, larger seeds), whereas there is no diminution of the level of MNT expression (page 45).

Finally, example 12c (pages 56 and foll.) indicates that another gene may be more suited than *mnt* for producing fertile plants with larger seeds. Indeed, using the IPT1 gene allows obtaining plants with normal vegetative development which produced large seeds.

The inventor of WO 2005/085453 is also the corresponding author of Hughes et al (Plant Biotechnol J. 2008 Oct;6(8):758-69. Epub 2008 Jul 8). This article further describes the *mnt* mutants of WO 2005/085453. The *mnt* gene is now correctly named *arf2.*

This article confirms that the *arf2* mutants have some kind of infertility. It also indicates that neither heterozygous nor homozygous mutants show any increase in yield. These heterozygous or homozygous mutants actually show a decrease in yield. In order to correct infertility problems, the authors engineered plants with a targeted expression of the *arf2* gene in sepals and petals to restore the opening of the flowers and the number of seeds.

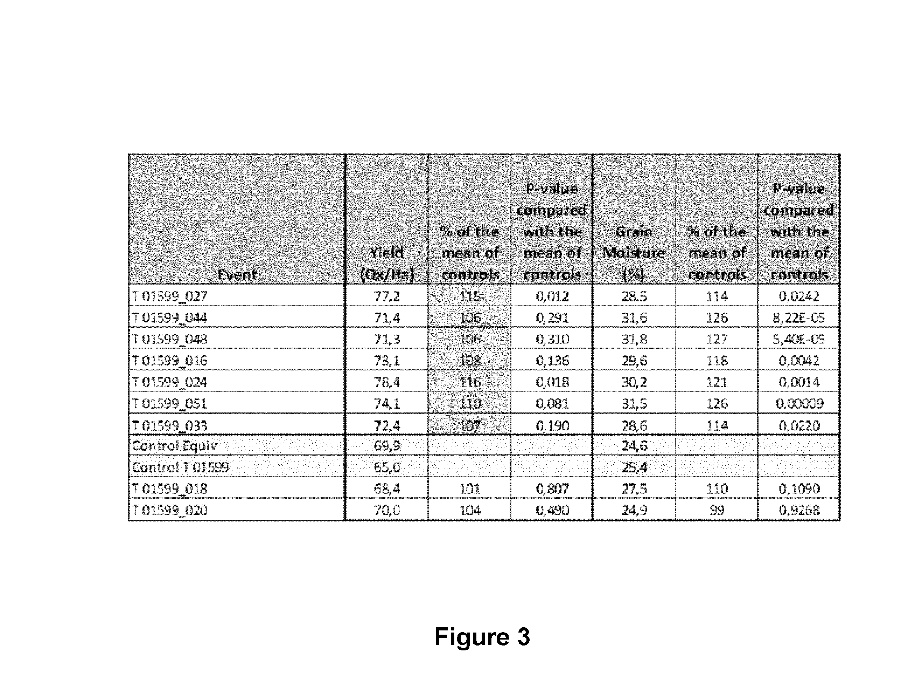

The authors observed that homozygous plants with the restoration of function in these organs have larger seeds than wild. However, the yield is lower (Figure 3c) due to the production of fewer seeds per plant. (page 763, right column). The authors calculated the Harvest Index, which is also reduced.

In conclusion, this article indicates that the *arf2* gene mutants have larger seeds, but that infertility is observed in the homozygous mutants (because of problems of flowering), and that yield is decreased for all tested plants (homozygous mutants, heterozygous mutants or homozygous mutants with partial restoration of the gene in the flowers to increase fertility).

Okushima et al (Plant J. 2005 Jul;43(1):29-46) describe three *arf2* mutants, with a number of phenotypic characteristics such as delay in flowering, flowers abnormal sterility as is reported in WO 2005/085453.

Analysis of the presence of the polypeptide shows that there is no protein production for the *arf2-8* mutant *arf2,* while there is accumulation of truncated polypeptide for *arf2-6* and *arf2-7* mutants (figure 5.e).

The authors mention that the seeds of the mutants are larger than wild plants (page 35, Figure 3.i). However, no conclusion is given on performance.

The authors also produced RNAi and overexpressing transgenic plants: they indicate that overexpression results in co-suppression, and neither the RNAi nor overexpressors (co-deleted) produce polypeptides. The phenotypes are similar to those of mutants. The results obtained on the overexpressor plants are similar to the ones reported in WO 2005/085453 with regards to the phenotype of the plants, but opposite with regards to the expression of the ARF2 protein.

Lim et al (J Exp Bot. 2010 March; 61(5): 1419-1430) performed EMS and identified the *arf2* gene as being mutated in plants which have delayed senescence of leaves. These plants also have larger aerial organs, inhibition of the opening of buds, delayed flowering, and delayed senescence of roots. The authors provide no information on seed size or yield.

Article Schruff et al (Development. 2006 Jan;133(2):251-61. Epub 2005 Dec 8.) also comes from the inventor of WO 2005/085453. This article describes the *mnt Arabidopsis* mutant which has seeds with larger size weight. As already described this mutant also present other phenotypes characteristics such as problem for opening buds and self-pollinating.

Vert et al (Proc Natl Acad Sci USA. 2008 Jul 15;105(28):9829-34. Epub 2008 Jul 3.) indicate that *arf2* mutants show a number of growth defects.

Unexpectedly, the inventors have demonstrated that it is possible to increase yield in cereals, by inhibiting (down-regulating) the *arf2* gene in these plants, without observing the sterility defects reported in the prior art.

In the context of the present invention, the *arf2* gene is the gene that codes for an Arf2 protein (which is an orthologue of *Arabidopsis thaliana* protein depicted in SEQ ID N° 28), wherein alleles of said Arf2 protein are depicted by SEQ ID N° 1 (for maize), SEQ ID N° 3 (for rice), SEQ ID N° 4 or SEQ ID N° 27 (for barley), SEQ ID N° 5 (for sorghum), SEQ ID N° 6 (for *Brachypodium)* and SEQ ID N° 7 to SEQ ID N° 9 (which may correspond to the Arf2 proteins for the three genomes of wheat).

It is known that the sequence of the *arf2* gene or of the Arf2 protein may vary within one species as there are allelic variations of the sequences for the same gene between different individuals in the same species.

Consequently, the Arf2 protein also encompasses proteins having a sequence presenting at least 95 % identity, more preferably at least 98 % identity, most preferably at least 99 % identity with SEQ ID N° 1, SEQ ID N° 3, SEQ ID N° 4, SEQ ID N° 27, SEQ ID N° 5, SEQ ID N° 6, SEQ ID N° 7, SEQ ID N° 8 or SEQ ID N° 9.

The degree of identity is defined by comparison with the entire sequence of reference. This percentage of identity is preferably obtained by the method of Needleman and Wunsch (J. Mol. Biol. 48: 443 (1970)).

"Percentage of sequence identity" can be determined by comparing two optimally aligned sequences over a comparison window, where the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

A preferred method uses the algorithm of Needleman and Wunsch.

In maize, said Arf2 protein is coded by the *arf2* gene, the cDNA sequence of which is represented by SEQ ID N° 2.

cDNA sequences coding for the other Arf2 proteins depicted in SEQ ID N° 3 to SEQ ID N° 9 and SEQ ID N° 27 can easily be found by a person skilled in the art on public databases, using the tblastn software.

In a first aspect, the invention relates to a cereal comprising at least one cell which presents total or partial inhibition of the expression of a gene coding for the Arf2 protein as specified above. Said inhibition is due to the presence of a "determinant" (as described below).

As foreseen in the present invention, a total inhibition of a gene coding for the Arf2 protein in a cell indicates either that:
(i) No *arf2* mRNA is detected in said cell after RNA isolation and reverse transcription or Northern Blot. In particular, total inhibition is obtained when no mRNA is detected after RNA isolation and reverse transcription according to the method described in the examples (which illustrates the method for maize).
(ii) No functional protein is produced in said cell. No functional protein is produced in absence of *arf2* mRNA (see (i)). In other cases, mRNA may be present but leads to the production of a truncated protein (as an illustration when the arf2 mRNA is incomplete, in particular in case of the presence of a mutation within the gene, in an intron or in an exon). Truncated proteins can be detected by isolation of the proteins, Western Blot and detection of the size of the protein with an antibody (polyclonal or monoclonal) directed against the Arf2 protein. Okushima *et al.* (*op. cit.*) describe such strategy and the means to obtain such data. Presence of mutations in the active site of the protein (binding site of the protein to DNA, as described in Vert et al (op.cit.)) may also lead to production of a non functional protein. Wang et al (Mol Biol Rep, 2011, Jun 11. [Epub ahead of print]) also describe precisely the structure of the ARF proteins, and the localization of the DNA binding domain.

As foreseen in the present invention, a partial inhibition of a gene coding for the Arf2 protein in a cell indicates *arf2* mRNA is detected in said cell after RNA isolation and reverse transcription or Northern Blot, but at a lower level than that detected in a cell which do not bear the determinant leading to *arf2* inhibition. In particular, partial inhibition is obtained when a lower level of mRNA is detected after RNA isolation and reverse transcription according to the method described in the examples (which illustrates the method for maize). In particular, partial inhibition is obtained when the level of *arf2* mRNA is lower than 0.9 times, more preferably lower than 0.75 times, and more preferably lower than 0.66 times of the level of arf2 mRNA in a cell which does not bear the determinant leading to *arf2* inhibition (the term "determinant" is described below). Preferably, said cell which does not bear the determinant leading to *arf2* inhibition is from a plant that is isogenic to the plant from which originates the cell in which partial inhibition is to be detected. Preferably, the cells are from the same plant tissue and mRNA is isolated at the same level of development. It is indeed most preferred that the level of inhibition is compared from comparable cells, only differing from the presence or absence of the determinant.

The level of *arf2* mRNA can be measured as an absolute level. It is nevertheless preferred that the level of *arf2* mRNA is measured as a relative level, compared to other control genes. In this case the method to be used to measure the level of mRNA and to detect inhibition is as follows:
- mRNA is isolated from tissues in which it is supposed to be inhibited and control tissues (such as leaves as an example)
- Reverse transcription and real-time quantitative PCR are performed on said mRNA using primers that amplify the *arf2* gene or primers that amplify control genes. These control genes are genes which are known to be usable as control in Northern Blot analysis, as their quantity level rarely varies. One can cite actin, ubiquitin 2, EF1α genes. It is preferred that at least two control genes are used, and in particular ubiquitin 2 and EF1 α.
- The Cp is then calculated for each amplified sample according to methods known in the art for real-time qPCR. In particular, machine used to perform real-time qPCR usually present a software which can automatically calculate this value, by calculation of the second derivative maximum.
- One then calculates the value equal to 2exp (-(Cp for *arf2* mRNA - Cp for control gene (or mean of Cp of the control genes). This value gives a relative level of expression for *arf2* mRNA as compared to the level of expression of the control gene. If this value is higher than 1, this means that there is more *arf2* mRNA than the control gene. If this value is lower than 1, this means that there is less *arf2* mRNA than the control gene.
- One then compares the values obtained for the cells in which *arf2* is inhibited (i.e. where the determinant is present) and for the cells in which *arf2* is at the basal level (i.e. where the determinant is absent). Ratio between these two values allows the determination of the level of inhibition of the *arf2* gene.

In a preferred embodiment, said cereal is maize (also named corn in this document). In this case, the sequence of one allele of an Arf2 protein is represented by SEQ ID N° 1. It is to be noted that this protein is also named Arf29 in maize (Wang, 2011 *op. cit*.).

In a specific embodiment, said cereal also presents inhibition of the expression of the *arf13* gene. In maize, the sequence of one allele of an Arf13 (according to the nomenclature of Wang) protein is represented by SEQ ID N° 10. The cDNA is represented by SEQ ID N° 11. It is clear that SEQ ID N° 10 represents the sequence of an allele of the Arf13 protein, and that the invention also covers inhibition of other alleles of the *arf13* gene, which code for proteins having a sequence presenting at least 95 % identity, more preferably at least 98 % identity, most preferably at least 99 % identity with SEQ ID N° 10.

These two maize sequences (ZmARF29 and ZmARF13, numbered according to the nomenclature of Wang) are putative *Arabidopsis thaliana* ARF2 protein orthologues.

Due to the similitude between Arf29 and Arf13 in maize, inhibition of both genes may occur depending whether the RNAi construct possesses long enough regions capable of hybridizing with both genes.

In a specific embodiment, said *arf2* gene is inhibited in multiple cells of said cereal, wherein said inhibition in multiple cells results in an inhibition in one or multiple tissues of said cereal. In this embodiment, it is possible that the *arf2* gene is not inhibited in other tissues of said cereal.

In another embodiment, said *arf2* gene is inhibited in all cells of said cereal. In this case, the invention encompasses a cereal which presents an inhibition of the expression of the *arf2* gene.

It is to be noted that the invention envisages that the inhibition of the *arf2* gene is obtained by various methods, such as mutation of the gene or transformation of the plant with a vector that will eventually cause the inhibition.

As indicated above, the inhibition is obtained by the introduction of a "determinant" in cells of said cereal. As foreseen herein, a "determinant" causes the inhibition of the *arf2* gene, is inheritable from generation to generation and is transmissible to other plants through crosses. Determinants will be described in more details below and include mutations and transgenes (introduced foreign DNA within the genome of the cells of the plant).

As indicated above, the inhibition may be said to be "total" or "full" (i.e. there is no more production of functional Arf2 protein) or "partial" (i.e. there is a decrease in the production of functional Arf2 protein, as compared with the production in a plant that does not contain the mutation or the vector causing the inhibition). It is also to be noted that inhibition does not preclude production of non-functional Arf2 protein (such as a truncated protein, in particular in case of a non-sense mutation present in the *arf2* gene).

It is also possible that the cereal presents a total inhibition of the *arf2* gene in some tissues, whereas there is no or only a partial inhibition in other tissues.

In an embodiment inhibition of the Arf2 expression is obtained by a mutation of the *arf2* gene through insertion of a transposable element or of a T-DNA or following physical mutagenesis.

In this embodiment, expression and/or activity of ARF2 is inhibited by mutagenesis of the gene coding for said protein.

The mutagenesis of the gene can take place at the level of the coding sequence or of the regulatory sequences for expression, in particular of the promoter. It is, for example, possible to delete all or part of said gene and/or to insert an exogenous sequence.

By way of example, mention will be made of insertional mutagenesis: a large number of individuals derived from a cereal that is active in terms of the transposition of a transposable element (such as the AC or Mutator elements in maize) are produced, and the cereals in which there has been an insertion in the *arf2* gene are selected, for example by PCR.

It is also possible to introduce one or more point mutations with physical agents (for example radiations) or chemical agents, such as EMS or sodium azide treatment of seed, site-directed DNA nucleases or gamma irradiation. The consequences of these mutations may be to shift the reading frame and/or to introduce a stop codon into the sequence and/or to modify the level of transcription and/or of translation of the gene. In this context, use may in particular be made of techniques of the "TILLING" type (Targeting Induced Local Lesions IN Genomes; McCALLUM et al., Plant Physiol., 123, 439-442, 2000). Such mutated cereals are then screened, in particular by PCR, using primers located in the target gene. One can also use other screening methods, such as Southern Blots or the AIMS method that is described in WO 99/27085 (this method makes it possible to screen for insertion), by using probes that are specific of the target genes, or through methods detecting point mutations or small insertions / deletions by the use of specific endonucleases (such as Ce/ I, Endo I, which are described in WO 2006/010646).

In this embodiment, the determinant as mentioned above is the mutation. It is indeed inheritable and transmissible by crosses.

In another embodiment, inhibition of the Arf2 expression is due to the presence in cells of said cereal of an antisense, or overexpression (leading to co-suppression), or RNAi construct. The DNA constructs used in these methods are introduced in the genome of said cereal through methods known in the art.

The transformation of vegetable cells can be achieved by any one of the techniques known to one skilled in the art.

In particular, it is possible to cite methods of direct transfer of genes such as direct micro-injection into plant embryos, vacuum infiltration or electroporation, direct precipitation by means of PEG or the bombardment by gun of particules covered with the plasmidic DNA of interest.

It is preferred to transform the cereal with a bacterial strain, in particular *Agrobacterium,* in particular *Agrobacterium tumefaciens.* In particular, it is possible to use the method described by Ishida et al. (Nature Biotechnology, 14, 745-750, 1996) for the transformation of Monocotyledons.

In particular, inhibition may be obtained by transforming the cereal with a vector containing a sense or antisense construct. These two methods (co-suppression and antisense method) are well known in the art to permit inhibition of the target gene. It is to be noted that the data obtained in *Arabidopsis* demonstrates that the co-suppression approach seems adapted for this gene.

One can also use the RNA interference (RNAi) method, which is particularly efficient for extinction of genes in plants (Helliwell and Waterhouse, 2003). This method is well known by the person skilled in the art, and comprises transformation of the cereal with a construct producing, after transcription, a double-stranded duplex RNA, one of the strand of which being complementary of the mRNA of the target gene.

The invention thus relates to a cereal, which is a cereal transformed with a transgene that comprises DNA construct comprising a polynucleotide chosen among:
a) a polynucleotide coding for the *arf2* gene as specified above ;
b) a polynucleotide which has a sequence complementary of the sequence of the polynucleotide of a) ;
c) a fragment of at least 12 consecutive nucleotides of a polynucleotide of a) or b), more preferably at least 20, even more preferably at least 50, the most preferably at least 100 consecutive nucleotides of a polynucleotide of a) or b), or capable of selective hybridization with a polynucleotide of a) or b) ;
d) a polynucleotide containing (i) a polynucleotide X comprising at least 12 nucleotides, preferably at least 15, advantageously at least 20, and even more preferably at least 50, most preferably at least 100 nucleotides capable of selective hybridization with the polynucleotide of a), and (ii) a polynucleotide Y that is complementary of said polynucleotide X,
under transcriptional control of an appropriate promoter.

Polynucleotides a) and some polynucleotides c) are suitable for inhibition by co-suppression. Polynucleotides b) and some polynucleotides c) are suitable for inhibition by antisense. Polynucleotides d) are suitable for inhibition by RNAi.

Said DNA constructs preferably comprise a nucleotide chosen from SEQ ID N° 12, SEQ ID N° 13, SEQ ID N° 14, SEQ ID N° 15 and SEQ ID N° 16. These nucleotides are in particular used for generating vectors suitable for RNAi in maize, wheat, rice, sorghum and brachypodium, respectively.

In a preferred embodiment, the cereal transformed with said DNA construct may contain said polynucleotide within its genome. In another embodiment, the transgene may have been introduced such as being expressed transiently, or through a genetic construct not integrated in the genome. Thus, agro-infiltration or any other methods, such as injection or spray, are contemplated for transient expression.

In this embodiment, the determinant as mentioned above is the DNA construct. It is to be noted that it is not necessarily present at the same locus than the *arf2* gene.

It is foreseen that said nucleic acids which are in the constructs are transcribed. They are thus under the control of an appropriate promoter. One can use various promoters, among which a constitutive, tissue specific, developmentally regulated, inducible promoter.

In a preferred embodiment, said construct is under the control of a constitutive promoter. In a most preferred embodiment, said construct is a RNAi construct, under the control of a constitutive promoter.

Other suitable promoter could be used. It could be a tissue-specific promoter such as a root-specific promoter, a leaf-specific promoter, a seed-specific, a BETL specific promoter and the like. Numerous tissue-specific promoters are described in the literature and any one of them can be used.

Examples of constitutive promoters useful for expression include the 35S promoter or the 19S promoter (Kay et al., 1987, Science, 236 :1299-1302), the rice actin promoter (McElroy et al., 1990, Plant Cell, 2 :163-171), the pCRV promoter (Depigny-This et al., 1992, Plant Molecular Biology, 20 :467-479), the CsVMV promoter (Verdaguer et al., 1998, Plant Mol Biol. 6:1129-39), the ubiquitin 1 promoter of maize (Christensen et al., 1996, Transgenic. Res., 5 :213), the regulatory sequences of the T-DNA of *Agrobacterium tumefaciens,* including mannopine synthase, nopaline synthase, octopine synthase.

One can also cite the promoters regulated during seed development such as the HMWG promoter (High Molecular Weight Glutenin) of wheat (Anderson O. D. et al., 1989, Theor Appl Genet, 77 : 689-700 ; Roberts et al., 1989, Plant cell, 1 :569-578), the waxy, zein or bronze promoters of maize.

Promoters may come from the same species or from another species (heterologous promoters). Although some promoters may have the same pattern of regulation when there are used in different species, it is often preferable to use monocotyledonous promoters in monocotyledons and dicotyledonous promoters in dicotyledonous plants. Consequently, the promoter is preferably a promoter that comes from a monocotyledonous plant, and more preferably from a cereal.

In a preferred embodiment, the promoter is the pCSVMV promoter which is represented by SEQ ID N° 17. It is within the scope of the invention to use a sequence that may vary from SEQ ID N° 17, for the same purpose (obtaining constitutive expression of a construct). Indeed, it is possible to add, delete or change some bases of SEQ ID N° 17, without modifying the pattern or level of expression of the promoter.

In preferred embodiments said construct comprises a RNAi construct, the sequence of which is depicted by SEQ ID N° 18. One can replace the specific maize sequence in SEQ ID N° 18 (sense from 1132 to 1881, and antisense from 3156 to 3897) by any of the sequences SEQ ID N° 13, SEQ ID N° 14, SEQ ID N° 15 or SEQ ID N° 16 in order to obtain a RNAi construct usable in wheat, rice, sorghum and *Brachypodium* respectively.

In SEQ ID N° 18,
- nucleotides 6 to 507 represent the CSVMV promoter
- nucleotides 411 to 417 represent the TATA box
- nucleotide 445 represent the beginning of the Open reading Frame
- nucleotides 544 to 1105 represent the rice actin intron
- nucleotides 1132 to 1923 represent the first part of the RNAi construct
- nucleotides 2009 to 2900 represent the rice tubulin intron
- nucleotides 3156 to 3947 represent the second part of the RNAi construct
- nucleotides 4024 to 4532 represent the AtSac66 terminator.

It is clear that the cereals used in the methods described below are cereals according to the invention, in which the gene *arf2* (or the production of the Arf2 protein) is inhibited, either totally or partially, in all cells or in specific tissues, totally in some tissues whereas not at all or only partially in other tissues, or with the same level of inhibition in all tissues.

These cereals are obtained as described above. They can easily be characterized using methods known in the art. One can cite RNA extraction and Northern Blot which makes it possible to determine an inhibition in the level of *arf2* mRNA. RNA extraction may be performed tissue by tissue and/or at various stages of development of the cereal, which makes it possible to determine the pattern of inhibition in particular when RNAi is used, with promoter having a specific pattern of expression (whether localized in specific tissues, or at different stages of development).

Alternatively, one can extract the whole protein content and perform a Western Blot in order to reveal the presence, absence or quantity of Arf2 protein. As usual, the quantity of Arf2 protein is preferably determined by comparison with the quantity of Arf2 protein in the same tissue of an isogenic plant which does not contain the determinant causing *arf2* inhibition.

In the most preferred embodiment, the cereal according to the invention are such that the *arf2* gene is partially inhibited, due to the presence of a transgene (DNA construct) within the cells of said cereal, which induces partial inhibition through antisense, co-suppression or RNAi methods, and wherein said transgene is under the control of a constitutive promoter.

The invention also relates to various methods of using the cereals of the invention.

In particular, the invention encompasses a method for improving yield in a cereal, comprising inhibiting the expression of a gene coding for the Arf2 protein as specified above in said cereal. In this method, the cereal with the inhibited Arf2 gene presents a higher yield than a second cereal not having an inhibited Arf2 gene, after sowing and harvest. In this method, it is clear that the increase of yield is checked by sowing and harvesting of a multiplicity of cereals that present inhibition of the *arf2* gene, the yield of which is then compared with the yield obtained with a second group of cereals not presenting said inhibition, and this under the same culture conditions (sowing and harvest at the same time, on comparable parcels, use of the same amount of fertilizers, water...). It is also clear that comparison is to be performed on a second group of cereals that is isogenic to the cereals having the inhibited *arf2* gene. This "isogenic" cereal differs from the cereal having an inhibited *arf2* gene at very few loci (less than 20, more preferably less than 10), and does not carry the determinant leading to inhibition of the *arf2* gene (said determinant being the mutation in the *arf2* gene (coding DNA or regulatory sequences) or the construct leading to inhibition of expression of the gene or protein). This cereal can also be called "virtually isogenic".

The invention also relates to a method for producing a cereal that can be used in a selection process for obtaining a cereal with improved yield, comprising the step of inhibiting the expression of a gene coding for the Arf2 protein in said cereal. The inhibition of the *arf2* gene can be performed by any method as described above.

Indeed, it is often preferable to sow and harvest cereals lines that have been optimized, in particular for the location in which they are cultured. Consequently, one will perform the genetic modifications (mutations, introduction of foreign DNA) in order to obtain a material that is then used for selection process. The eventual lines are obtained by introgressing the determinant leading to inhibition of the *arf2* gene in reference lines having otherwise agronomic quality characteristics.

The introgression of the characteristic is in particular carried out by selection, according to methods known in the art (crossing and self-pollination). The plants are in particular selected using molecular markers.

The principle is recalled below:
A series of back crosses are performed between the elite line (in which one wishes to introduce the determinant) and a line that already carries said determinant (the donor line). During the back crosses, one can select individuals carrying the determinant and having recombined the smallest fragment from the donor line around the determinant. Specifically, by virtue of molecular markers, the individuals having, for the markers closest to the determinant, the genotype of the elite line are selected.

In addition, it is also possible to accelerate the return to the elite parent by virtue of the molecular markers distributed over the entire genome. At each back cross, the individuals having the most fragments derived from the recurrent elite parent will be chosen.

The invention thus also encompasses a method for selecting a cereal comprising the step of
a) selecting, in a population of cereals, the cereals in which the *arf2* gene is inhibited.

Said selected cereal is suitable to be used, and can later be used in a selection process for obtaining a cereal with improved yield.

In a specific embodiment, said population of cereal is the progeny obtained from a cross between a first cereal line in which the *arf2* gene is inhibited and a second cereal line in which the *arf2* gene is not inhibited.

Said inhibition of the *arf2* gene is caused by the presence of a determinant (mutation or foreign DNA as described above) within the genome of the cereal. Said selection in step a) is thus performed by identification, in the genome of said cereal, of the presence of said determinant causing *arf2* inhibition (either directly through analysis of the genomic DNA of the cereal, or indirectly through analysis of the presence or absence of the products that should be obtained from the *arf2* gene (mRNA from the *arf2* gene and/or presence or absence of a functional or truncated Arf2 protein)).

In a specific embodiment, the selection in step a) is performed through the use of a marker that is specific to the determinant leading to the inhibition of said *arf2* gene. In this embodiment, step a) is thus preferably preceded by a step comprising genotyping said population of cereals.

In a specific embodiment, the selection in step a) is preceded by a step comprising extracting the RNA from the individuals in said population and performing a Northern Blot (or an equivalent method) in order to identify the cereals in which the production of mRNA from the *arf2* gene is inhibited. RNA may be extracted from specific tissues only.

In a specific embodiment, the selection in step a) is preceded by a step comprising extracting proteins from the individuals in said population and performing a Western Blot (or an equivalent method) in order to identify the cereals in which the production of Arf2 protein is inhibited. Protein may be extracted from some specific tissues only.

The invention also comprises a method for obtaining a cereal exhibiting increased yield, comprising the step of introgressing the determinant responsible for the inhibition of the *arf2* gene into said cereal.

This method comprises the steps consisting in
a) crossing a first cereal line that has said determinant with a second cereal that does not have said determinant,
b) selecting, in the progeny obtained, the cereals in which the *arf2* gene is inhibited and which present the best genome ratio as regards said second cereal
c) performing a back cross of said selected cereals with said second cereal line
d) repeating, if necessary, steps b) and c) until an isogenic line of said second cereal, and containing said determinant, is obtained,
e) optionally, performing a self-pollination in order to obtain a cereal that is homozygous for said determinant.

The invention also relates to a method for increasing a cereal yield for a cereal harvest, comprising the step of sowing cereal seeds, wherein said cereal seeds grow into plants that exhibits an inhibited expression of an *arf2* gene, and wherein the yield of the harvested cereals is increased as compared to the yield obtained from harvested isogenic cereals which do not exhibit said inhibited expression of *arf2* gene.

If necessary, the comparison may be performed as mentioned above.

The invention also relates to a method of growing cereals, comprising the step of sowing the cereals of the invention, and growing cereals from the sowed seeds.

The invention may also comprise the step of harvesting said cereals.

The invention also relates to a method for harvesting cereals comprising the step of harvesting cereals of the invention.

It is clear that the cereals used in the methods described below are cereals according to the invention, in which the gene *arf2* (or the production of the Arf2 protein) is inhibited, either totally or partially, in all cells or in specific tissues, totally in some tissues whereas not at all or only partially in other tissues, or with the same level of inhibition in all tissues, as described above.

### FIGURES

**Figure** 1: schematic description of plasmid pBIOS1375, used to trigger RNAi in maize. The two inverted and complementary *arf2* fragments needed for RNAi are indicated (ZmARF2-like partial) and are separated by a tubulin intron from rice. This RNAi fragment is driven by the CsVMV promoter. RB, LB: Right Border, Left Border; OsTUBL intron: Tubulin intron from rice; pro Actin: rice actin promoter; +1: start of open reading frame; 3'Nos: terminator; cos Lambda: zone of recombinaison used for making the plasmid; 3'AtSac66: terminator
**Figure** 2: relative level of mRNA production from T0 plants, normalized with regards to the mean of mRNA levels for EF1a and ubiquitin (see example 2). The transformation events chosen for field trial are indicated by an arrow.
**Figure 3****:** Table summarizing the field trial results for various transformation events (see example 3).

### EXAMPLES

### Example 1 - Generation of vectors intended to perform RNAi on the arf29 gene in maize, with pCSVMV

### Cloning.

Step 1: The ZmARF2 like fragment (SEQ ID N° 12) was amplified from genomic DNA from leaf tissue of the inbred line J1A (A188) by PCR using the following primers,
OI 1545 ZmARF2_OI5: CACATCAAAATTCGGTCTTGCAAA (SEQ ID N° 19)
OI 1546 ZmARF2_OI6 : TTCCAGCTGTGCTTACGGACA (SEQ ID N° 20)
and then subcloned into pGEMT-Easy (Promega) forming pBIOS1351.

Step 2: The Spel blunt / Notl fragment (A188 partial ZmARF2-like) from pBIOS1351 was introduced into pENTR1A (Invitrogen) opened with Xmnl and Notl forming pBIOS1363 "ARF2-like Entry clone".

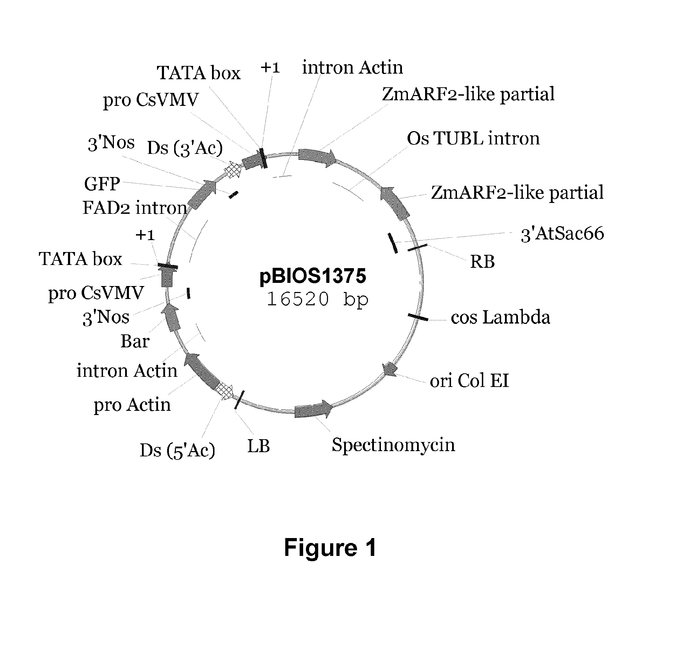

Step 3: the binary plasmid pBIOS1375 (Figure 1) was obtained by GATEWAY "LR Reaction" between pBIOS1363 "ARF2-like Entry clone" and pBIOS 893 the "Destination vector". pBIOS 893 is a derivative of pSB11 (Komari et al (1996), Plant J. 10, 165-74) containing a pActin-Bar gene for selection of maize transformants.
pBIOS1375 was used for maize transformation according to example 2.

### Example 2 - Transformation and characterization of transformed plants with regards to the Arf29 expression

### Transformation of maize plants with Agrobacterium tumefaciens carrying the RNAi construct pBIOS1375

The transformation is effected via the transformation protocol of Ishida et al. (Nature Biotechnology, 14, 745-750, 1996) for transformation with *A. tumefaciens.*

Immature cobs of the A188 maize line produced under glass are taken 10 days after pollination and sterilized for 15 mins. The embryos are taken and placed in contact with a suspension of *Agrobacterium* containing the above described pBIOS1375 vector for 5 mins. After removal from the suspension of *Agrobacterium,* the embryos are cultured on a medium containing neither bacteriostatic nor selective agent. This co-culturing takes place in the dark for 4 to 7 days. After the co-culturing, the embryos are pricked out again on a fresh callogenesis medium containing the bacteriostatic and the selective agent. A callus initiates and develops from the transformed cells of these embryos. The callogenesis step takes place at 25°C in the dark and lasts 5 weeks. The callus embryos are picked out again on fresh medium every 2 to 3 weeks. At the end of this stage, the calluses are pricked out again on a regeneration medium for 5 weeks with repicking of the callus onto fresh medium after 2 to 3 weeks, Regeneration of plantlets is effected from the calluses, which are subjected to rooting in tubes once they are sufficiently developed. After 10-15 days in tube, the plantlets are acclimatized in a phytotron before being transferred to the greenhouse.

### Preparation of RNA

Total RNA of the different samples was extracted from leaves of transformants at the T0 stage using the SV96 Total RNA Isolation System Protocol, according to the recommendations of the manufacturer (Promega).

To check the quality of RNA, a measure of Optical Density (OD) was conducted by spectrophotometry to estimate the amount and the purity of the obtained RNA, by measurement of the ratio of 260/280 (which must be higher than 1.8). An aliquot of RNA was used to verify the absence of contamination in DNA by quantitative PCR. In addition the RNA integrity was verified by capillary electrophoresis LabChip GXII (Caliper).

### cDNA synthesis

500 ng of RNA are reverse-transcribed with random hexamers in a final volume of 20 µl according to the High Capacity cDNA archive protocol by following the recommendations of the supplier (Applied Biosystems).

### Primer Design

Primer design was performed on the Arf2 sequence (GenBank accession number: BT067605) with the Primer Express v2.0 Software (Applied Biosystems) outside the area targeted by RNAi, and being as far as possible in the 3' region. Arf2 gene was amplified with the forward primer 5'-AGCCTTCTACTATCCCGCGG-3' (SEQ ID N° 21) and reverse primer 5'-AGGCTCTATCTTCCAGGGAGAGAC-3' (SEQ ID N° 22) on a range of 5 points of 10 times dilution of cDNA of A188 untransformed plants to calculate the PCR efficiency.

The alignment of these primers with different members of the Arf family shows that they are not specific of Arf 29, Arf 13 is therefore also amplified in the PCR.

EF1 α, and Ubiquitin 2 genes are used for normalization (EF1 α : forward primer 5'-TGGTGAAGATGATACCGACCAA-3' (SEQ ID N° 23), reverse primer 5'-AGCAACCGTCTGCCTCATGT-3' (SEQ ID N° 24), Ubiquitin 2 : forward primer 5'-TGCTGGACCTCGATTGGTTT-3' (SEQ ID N° 25), reverse primer 5'-GTCCTGCATCAACCGCTTG-3' (SEQ ID N° 26)).

### Real-time quantitative PCR and data analysis

2µl of a 12 times dilution of the cDNA are mixed with 3 µl primer of forward and reverse primers (1 µM each) and with 5 µl of the mix PCR containing sybrgreen Fast Start SYBR Green Master with ROX (Roche).

PCR is carried out in a volume of 10µl final on a LightCycler 480 (Roche) at 95 °C for 5 min followed by 40 cycles at 95 °C for 15 seconds and 60 °C for 45 sec and followed by a curve of fusion according to the recommendations of the manufacturer. The threshold Cycle (Cp) is determined in an automatic way by the software of the LC480 according to the method of calculation of the maximum of the second derivative.

The data are then analyzed in Excel®:
- calculation of the average of the normalization genes for each sample
- calculation of the dCp = Cp ARF2 - average Cp for normalization genes for each sample
- calculation of the value for the relative level of expression (2^{-dCp}) for each sample

A PCR on a non-transgenic sample (A188) was conducted to estimate the basal level of expression of the Arf2 gene. The value for the relative level of expression (2^{-dCp}) is 1.14. Consequently, all maize which harbors a relative level of expression lower than 1.14 presents an inhibition in the *arf2* gene.

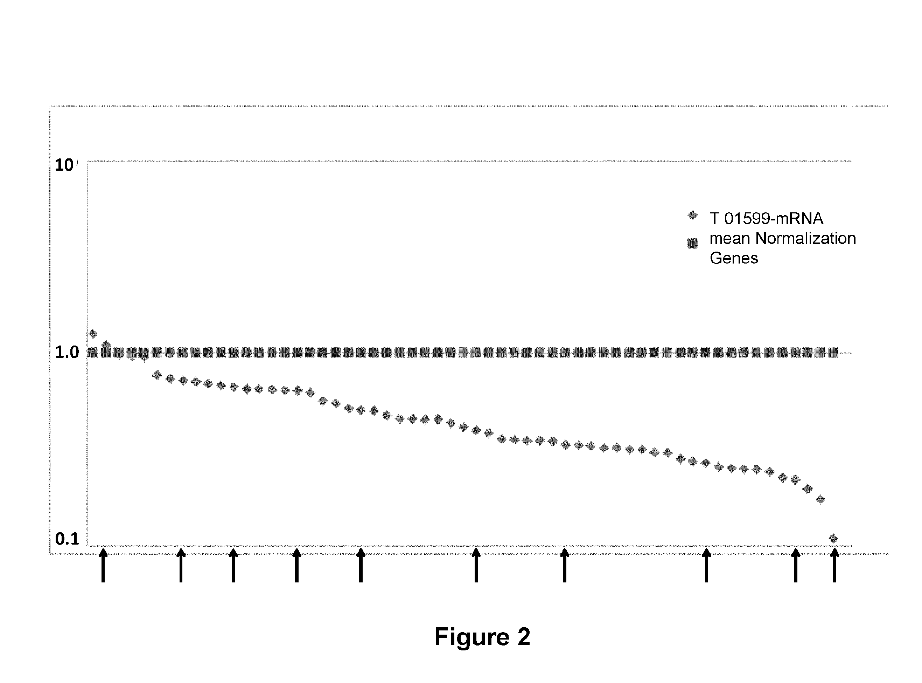

**Figure 2** describes the results obtained from different transformation events. It can be seen that the level of expression of the *arf2* gene varies depending on the transformation events. This is not surprising as it is known that the expression of a transgene depends on multiple factors (that are not well understood), such as the localization of the transgene with the plant genome. In any case, Figure 1 demonstrates that it is possible to obtain multiple transformants that harbour a reduction in the level of expression of *arf2* RNA.

Nine lines (indicated by an arrow on **Figure 2**) were chosen for further analysis and field trials, some of which presenting a high inhibition of the relative level of *arf2* mRNA (as compared to the A188 control), and others having a lower inhibition (see Table I).

**Table I - Relative level of arf2 mRNA compared to A188 for T0 plants**

| T0 Event | Relative level of *arf2* mRNA compared to A188 |
|---|---|
| T 01599_027 | 0.19 |
| T 01599_044 | 0.29 |
| T 01599_048 | 0.63 |
| T 01599_016 | 0.56 |
| T 01599_024 | 0.44 |
| T 01599_051 | 0.24 |
| T 01599_033 | 0.10 |
| T 01599_018 | 0.58 |
| T 01599_020 | 0.96 |

### Example 3 - Field trials

Hybrids with a tester line were obtained from T3 plants issued from the nine T0 ARF2 RNAi transgenics maize lines (CsVMV+Actlnt + ARF2 RNAi + Sac66 term) chosen according to example 2.

The transformants (T0) plants were first crossed with the A188 line thereby producing T1 plants. T1 plants were then self pollinated twice, producing T3 plants which are homozygous lines containing the transgene. These T3 plants were then crossed with the tester line thereby leading to an hybrid. This hybrid is at a T4 level with regards to the transformation step and is heterozygous for the transgene. These hybrid plants are used in field experiments.

Control hybrids are obtained as follows:
Control Equiv corresponds to a cross between A188 line (the line used for transformation) and the tester line.

Control T 01599 corresponds to a cross between a null segregant (isolated after the second self-pollination of the T1 plants) and the tester line. Said null segregant is an homozygous line which does not bear the transgene. Although the null segregant theorically presents the same genome as A188, it has undergone *in vitro* culture (via the steps of callus differentiation and regeneration) and may thus present mutations (either genetic or epigenetic) with regards to a A188 line that has not undergone *in vitro* culture.

These two control lines are used to avoid any effect that could be due to mutations (genetic or epigenetic) coming from *in vitro* culture.

Improved yield was observed for the hybrid plants containing the RNAi construct as compared to the controls, as can be seen in Figure 3.

It can also be seen that moisture is increased in RNAi transformed plants as compared to the controls.

Yield was calculated as follows:
During harvest, grain weight and grain moisture are measured using an on-board equipment on the combine harvester.

Grain weight is then normalized to moisture at 15 %, using the following formula: Normalize grain weight = measure grain weight x (100 - measured moisture (as a percentage)) / 85 (which is 100 - normalized moisture at 15 %).

As an example, if the measured grain moisture is 25 %, the normalized grain weight will be: normalized grain weight = measured grain weight x 75 / 85.

Yield is then expressed in a conventional unit (such as quintal per hectare).

Experimental design:
Plants were sown on May 18, 2010, and harvested On September 29, 2010.

The experimental block comprised 5 replicates. Each replicate comprised about 58.5 plants per plot at a density of 69600 plants/ha.

Two controls were used present in this experiment as described above (a null segregant T01599 and a control equivalent (A188 crossed with the tester line). Results are represented on figure 3, with the yield expressed in (Qx/ha).

This figure demonstrates that the transgenic plants present an increased yield (normalized for moisture) and an increased grain moisture compared to the control. No sterility abnormalities such as the ones described for the *arf2*-mutant, RNAi or co-suppressed *Arabidopsis* of the prior art were observed for these plants.

### Example 4

Other RNAi constructs can be designed for inhibition of the *arf2* gene in wheat, rice, sorghum or brachypodium, using respectively the sequences SEQ ID N° 13, SEQ ID N° 14, SEQ ID N° 15, SEQ ID N° 16 as sequences for the "sense" part of the RNAi construct.

## Claims

1. A cereal comprising at least one cell which presents inhibition of Arf2 expression, by inhibition of the *arf2* gene coding for the Arf2 protein, wherein alleles of said Arf2 protein are depicted by SEQ ID N° 1 (for maize), SEQ ID N° 3 (for rice), SEQ ID N° 4 or SEQ ID N° 27 (for barley), SEQ ID N° 5 (for sorghum), SEQ ID N° 6 (for Brachypodium) and SEQ ID N° 7 to SEQ ID N° 9 (corresponding to the Arf2 proteins for the three genomes of wheat).

2. The cereal of claim 1, which is maize, and wherein an allele of said Arf2 protein exhibit SEQ ID N° 1.

3. The cereal of any of claims 1 or 2, wherein said Arf2-coding gene is inhibited in specific tissue(s) of said cereal and is not inhibited in other tissues of said cereal.

4. The cereal of any of claims 1 to 3, wherein said inhibition of said Arf2-coding gene is a partial inhibition.

5. The cereal of any of claims 1 to 3, wherein said inhibition of said Arf2-coding gene is a total (full) inhibition.

6. The cereal of any of claims 1 or 5, wherein inhibition of the Arf2 expression is due to a mutation of the *arf2* gene through insertion of a transposable element or of a T-DNA or following physical mutagenesis.

7. The cereal of any of claims 1 or 5, wherein inhibition of the Arf2 expression is due to the presence, in the genome of said cereal of an antisense, or overexpression (leading to co-suppression), or RNAi construct.

8. The cereal of claim 7, wherein inhibition of the Arf2 expression is due to the presence, in the genome of said cereal, of a RNAi construct, wherein said construct is under the control of a constitutive promoter.

9. The cereal of claim 8, wherein said promoter is pCSVMV (represented by SEQ ID N° 17).

10. The cereal of any of claims 4 to 6, wherein said construct comprises a RNAi construct, the sequence of which is depicted by SEQ ID N° 18.

11. A method for improving yield in a cereal, comprising inhibiting the expression of the *arf2* gene coding for the Arf2 protein in said cereal, wherein said cereal with the inhibited arf2 gene presents a higher yield than a cereal not having an inhibited *arf2* gene.

12. A method for producing a cereal that can be used in a selection process for obtaining a cereal with improved yield, comprising the step of inhibiting the expression of the *arf2* gene coding for the Arf2 protein in said cereal.

13. The method of claim 12, wherein said inhibition is obtained by transformation of said cereal with an antisense, or overexpression, or RNAi construct, or by mutation of the *arf2* gene, through insertion of a transposable element or of a T-DNA or by physical mutagenesis.

14. A method for increasing cereal yield, comprising the step of sowing cereal seeds, wherein said cereal seeds grow into plants that exhibits an inhibited expression of the *arf2* gene coding for the Arf2 protein, and wherein the yield of the harvested cereals is increased as compared to the yield obtained from harvested cereals not exhibiting an inhibited expression of the *arf2* gene.

15. A method for selecting a cereal that can be used in a selection process for obtaining a cereal with improved yield comprising the step of selecting, in a population of cereals, the cereals in which the *arf2* gene is inhibited.
